# EUROPEAN PATENT APPLICATION

(11) **EP 4 567 592 A1**
(43) Date of publication of application: **11.06.2025**
(21) Application number: 24217467.0
(22) Date of filing: 04.12.2024
(51) Int. Cl.: G06F 9/445, G16H 40/40, G16H 40/63

(54) **METHODS AND SYSTEMS FOR MANAGING EXECUTION OF SURGICAL SOFTWARE APPLICATIONS**

(30) Priority: 05.12.2023 US 202363606575 P
(71) Applicant: Stryker Corporation, Portage, MI 49002 (US)
(72) Inventor: WOOLFORD, Brady Lewis, Portage, 49002 (US); ZEH, Christopher, Portage, 49002 (US)
(74) Representative: V.O.

(57) **Abstract**

An exemplary device for providing a plurality of surgical software applications comprises one or more processors; a plurality of surgical software applications for processing surgical data configured to be executed by the one or more processors; and one or more operating system programs for testing and providing the plurality of surgical software applications, and configured to be executed by the one or more processors. The one or more operating system programs include instructions for: receiving a user request to launch a surgical software application of the plurality of surgical software applications; retrieving one or more contract files associated with the surgical software application, comprising one or more parameters; comparing a current state of the device with the one or more parameters in the one or more contract files; and determining, based on the comparison, whether to launch the surgical software application.

## Description

### FIELD

The present disclosure relates to providing and managing surgical software applications.

### BACKGROUND

Surgical environments such as operating rooms can benefit from a dedicated electronic device that is configured to receive and process surgical data to guide surgical procedures and improve surgical safety. Such a dedicated electronic device can operate as a medical data processing device that receives various inputs associated with the surgical environment (e.g., surgical images, readings from medical devices in the operating room, commands from members of the surgical staff), launch one or more surgical software applications to process the inputs, and provide outputs (e.g., enhanced surgical images) to aid the surgical procedure.

However, many surgical software applications may impose a high computational burden on the electronic device and furthermore have operational constraints such that the electronic device may not provide sufficient resources to properly and safely execute one or more of the surgical software applications. For example, a surgical software application configured to enhance surgical videos may require a relatively high percentage of one or more processors (e.g., central processing unit (CPU), graphics processing unit (GPU)) and/or memory to output visual overlays in real time. As another example, a surgical software application may be compatible with only a specific version of an operating room and/or may require a specific hardware component. Thus, it would be desirable to provide a feature that allows a user (e.g., a surgeon, a nurse, a surgical staff member, a hospital administrator, a medical sales representative, a system administrator, a software developer, or the like) to determine whether a specific electronic device can properly and safely execute one or more surgical software applications without the occurrence of any undesirable events (e.g., a software crash event, a software hang event, a software lag event, a failure-to-launch event, an error, or the like) before a surgical procedure.

Further, due to the critical nature of the many surgical software applications, it is beneficial to continually monitor the execution of the surgical software applications on the electronic device to prevent the occurrence of undesirable events during a surgical procedure. An undesirable event may be triggered, for example, due to the launch of an additional surgical software application or due to the overheating of the electronic device. Thus, it would be desirable to provide features to monitor the electronic device (e.g., memory usage, processor usage, temperature) and to perform mitigation actions to prevent occurrence of undesirable events. Furthermore, it would be desirable to equip the electronic device with cooling mechanisms to dissipate heat effectively.

### SUMMARY

Disclosed herein are exemplary devices, apparatuses, systems, methods, computer program products, and non-transitory storage media for providing and testing a plurality of surgical software applications by utilizing sample input datasets. An exemplary device can comprise a plurality of surgical software applications for processing surgical data. Each surgical software application can include one or more sample input datasets that are configured to allow the surgical software to perform one or more of the software application's functionalities. The sample input dataset can comprise a variety of types of data, including image data, textual data, audio data, simulated user input data, or any combination thereof. The sample input dataset can be stored as a file or a collection of files (e.g., text file(s), audio file(s), or the like).

Using the sample input dataset(s), a user can test whether one or more surgical software applications can be safely and properly executed on the electronic device. For example, the device (e.g., one or more operating system programs of the device) can retrieve one or more sample input datasets corresponding to the one or more surgical software applications indicated by the user and execute, using the one or more sample input datasets, the one or more surgical software applications in a simulated environment. The device can then evaluate the execution of the one or more surgical software applications and output a recommendation related to the one or more surgical software applications. In some examples, the device outputs (e.g., via a display and/or speaker of the device) a recommendation to not execute the one or more surgical software applications on the device if an undesirable event has occurred during the simulated execution of the one or more surgical software applications. In some examples, the device outputs a recommendation to not execute the one or more surgical software applications on the device if a parameter related to the execution (e.g., memory usage, processor usage, or the like) exceeds a predefined resource threshold. In some examples, the recommendation includes one or more reasons that the one or more surgical software applications cannot be run together or suggested actions to resolve the issue (e.g., acquiring certain software/hardware component, making certain software/hardware component of the device available, terminating one or more surgical software applications that are currently running, or the like).

Also disclosed herein are exemplary devices, apparatuses, systems, methods, computer program products, and non-transitory storage media for performing pre-launch checks for a surgical software application. Upon receiving a user request to launch a surgical software application on a device, the device can first check the current state of the device and determine whether the current state of the device would allow the surgical software application to launch and operate properly. For example, each surgical software application of the device may include one or more corresponding contract files. The contract file(s) for each surgical software application may be or include one or more electronic documents that can specify a plurality of parameters, such as: a first set of parameters specifying an amount of resources required to execute the surgical software application, a second set of parameters specifying one or more hardware components required to execute the surgical software application, a third set of parameters specifying one or more compatibility constraints of the surgical software application, a fourth set of parameters specifying a relative priority of the surgical software application, or the like. Based on the current state of the device and the parameters specified in the contract file(s), the electronic device may determine to not launch the surgical software application. The electronic device may alternatively determine to terminate or throttle a currently executing surgical software application in order to launch the new surgical software application (e.g., based on their priority values).

Also disclosed herein are exemplary devices, apparatuses, systems, methods, computer program products, and non-transitory storage media for continuously monitoring a temperature associated with the device (e.g., via a temperature sensor inside the chassis of the device) and performing a series of mitigation actions if the temperature associated with the device exceeds a predefined temperature threshold to reduce the temperature of the device. The mitigation actions can include, for example, increasing the speed of a fan, enabling power saving modes on one or more processors, terminating one or more active surgical software applications (e.g., based on their priority values), or any combination thereof.

Also disclosed herein are cooling ducts for managing the temperature of an electronic device. The cooling ducts can include air vents to direct cooler air from outside the chassis of the device to processing units. The cooling ducts may be manufactured via 3D printing techniques. Manufacturing the cooling ducts via 3D printing can be more cost-efficient than molding, generate single pieces having complex geometries, and allow for quick updates to the designs due to changes to the processing units. Accordingly, the dimensions and shapes of the cooling ducts can be modified to be adapted to support different types of processing units to dissipate heat and reduce vibration, thus reducing the need for dedicated support brackets.

Also disclosed herein are exemplary devices, apparatuses, systems, methods, computer program products, and non-transitory storage media for providing a plurality of surgical software applications. An exemplary device can comprise one or more processors, and a plurality of surgical software applications for processing surgical data. The plurality of surgical software applications can be configured to be executed by the one or more processors. The exemplary device can comprise one or more operating system programs for testing and providing the plurality of surgical software applications. The one or more operating system programs can be configured to be executed by the one or more processors. The one or more operating system programs can include instructions for receiving a user request to launch a surgical software application of the plurality of surgical software applications, retrieving one or more contract files associated with the surgical software application, wherein the one or more contract files comprise one or more parameters, comparing a current state of the device with the one or more parameters in the one or more contract files, and determining, based on the comparison between the current state of the device with the one or more parameters in the one or more contract files, whether to launch the surgical software application.

The device may determine not to launch the one or more surgical software applications on the device if a parameter related to the execution (e.g., memory usage, processor usage, or the like) exceeds a predefined resource threshold. For instance, if the surgical software application cannot be launched without terminating or throttling one or more currently running software applications with lower priority values, the device can determine to not launch the surgical software application. The device may provide a notification to the user (e.g., a surgeon, a nurse, a surgical staff member, or the like) to terminate or throttle one or more currently running software applications with lower priority values. The device may provide a notification to the user that the device will automatically terminate or throttle one or more currently running software applications with lower priority values unless such automatic terminating or throttling is aborted by the user. Alternatively, the device may automatically terminate or throttle one or more currently running software applications with lower priority values, without user confirmation.

In accordance with determining to launch the one or more surgical software applications, the device may proceed with launching the one or more surgical software applications. Proceeding with launching the one or more surgical software applications may include a pre-launch check of one or more surgical software applications as described herein. Alternatively, the device may launch the one or more surgical software applications without a pre-launch check. The device may prompt a user to confirm whether to proceed with launching of the one or more surgical software applications, or the device may proceed with launching automatically, without user confirmation.

In accordance with determining not to launch, the device may not launch the one or more surgical software applications. The device may provide one or more notifications associated with the one or more surgical software applications not being launched, which may include a notification as described herein. Optionally, the device may prevent launch of the one or more surgical software applications such that a user cannot launch the one or more surgical software applications. Optionally, the device may prevent launch of the one or more surgical software applications until a notification has been acknowledged by a user. For example, the device may display a notification that the one or more surgical software applications failed the evaluation and will not be launched unless a user acknowledges and clears the notification.

Examples of the present disclosure provide numerous technical advantages. They allow a user (e.g., a medical practitioner, a hospital administrator, a medical sales representative, a system administrator, a software developer, or the like) to test whether one or more surgical software applications can be properly and safely executed on an electronic device before surgical procedures. The tests can allow the user to launch surgical software applications in an informed manner to ensure surgical safety during surgical procedures. The tests can also help the user to evaluate a newly installed surgical software application to ensure that the electronic device can provide the resources to properly execute the surgical software application. Further, examples of the present disclosure can reduce the occurrence of undesirable events (e.g., a software crash event, a software hang event, a software lag event, a failure-to-launch event, an error, or the like) before and during surgical procedures by conducting pre-launch checks of a surgical software application and effectively managing the temperature of the device. Accordingly, examples of the present disclosure can result in a system that is more flexible, robust, efficient, and economical than conventional systems.

An exemplary device for providing a plurality of surgical software applications comprises: one or more processors; a plurality of surgical software applications for processing surgical data, wherein the plurality of surgical software applications are configured to be executed by the one or more processors, and wherein the plurality of surgical software applications include a plurality of sample input datasets corresponding to the plurality of surgical software applications; and one or more operating system programs for testing and providing the plurality of surgical software applications, wherein the one or more operating system programs are configured to be executed by the one or more processors, and wherein the one or more operating system programs include instructions for: receiving one or more user inputs indicative of one or more surgical software applications of the plurality of surgical software applications; retrieving one or more sample input datasets corresponding to the one or more surgical software applications from the plurality of sample input datasets; executing, based on the one or more sample input datasets, the one or more surgical software applications in a simulated environment; evaluating the execution of the one or more surgical software applications; and outputting, based on the evaluation of the execution of the one or more surgical software applications, a recommendation related to the one or more surgical software applications.

In some examples, a sample input dataset of the plurality of sample input datasets comprises: image data, textual data, audio data, simulated user input data, or any combination thereof. In some examples, the sample input dataset of the plurality of sample input datasets comprises: surgical video data, medical record data, case file data, speech data, or any combination thereof. In some examples, evaluating the execution of the one or more surgical software applications comprises: identifying a software crash event, a software hang event, a software lag event, a failure-to-launch event, an error, or any combination thereof. In some examples, evaluating the execution of the one or more surgical software applications comprises: evaluating usage of one or more memories of the device or the one or more processors of the device.

In some examples, the one or more operating system programs further include instructions for: receiving a user request to launch a surgical software application of the plurality of surgical software applications; retrieving one or more contract files associated with the surgical software application, wherein the one or more contract files comprise one or more parameters; comparing a current state of the device with the one or more parameters in the one or more contract files; and determining, based on the comparison between the current state of the device with the one or more parameters in the one or more contract files, whether to launch the surgical software application.

In some examples, the one or more operating system programs further include instructions for: in accordance with a determination to not launch the surgical software application, outputting a user notification. In some examples, the one or more parameters in the one or more contract files comprise a first set of parameters specifying an amount of resource required to execute the surgical software application. In some examples, the first set of parameters is indicative of: a minimum memory requirement, a minimum core requirement, a minimum usage percentage, or any combination thereof. In some examples, the one or more parameters in the one or more contract files comprise a second set of parameters specifying one or more hardware components required to execute the surgical software application. In some examples, the one or more hardware components comprise: a central processing unit (CPU), a graphics processing unit (GPU), a capture card, an isolation port, a USB port, or any combination thereof. In some examples, the one or more parameters in the one or more contract files comprise a third set of parameters specifying compatibility constraints of the surgical software application. In some examples, the third set of parameters is indicative of: one or more software environments that the surgical software application is compatible with. In some examples, the one or more parameters in the one or more contract files comprise a fourth set of parameters specifying a relative priority of the surgical software application. In some examples, the relative priority is determined based on a relative risk level associated with a surgical procedure. In some examples, the fourth set of parameters is indicative of: a priority value of the surgical software application and/or whether the surgical software application can run without a displayed graphical user interface.

In some examples, at least one surgical software application of the plurality of surgical software applications is subscription-based.

In some examples, the device further comprises a chassis and a temperature sensor inside the chassis, and wherein the one or more operating system programs further include instructions for: obtaining, via the temperature sensor, a temperature associated with the device; if the temperature exceeds a predefined temperature threshold, reducing an amount of power provided to the one or more processors.

In some examples, the one or more operating system programs further include instructions for: if the temperature exceeds the predefined temperature threshold, terminating an active surgical software application being executed by the one or more processors. In some examples, the active surgical software application is selected based on a priority value associated with the active surgical software application and/or a last-used time associated with the active surgical software application.

In some examples, the device comprises a fan and wherein the one or more operating system programs further include instructions for: if the temperature exceeds the predefined temperature threshold, increasing a speed of the fan.

In some examples, the one or more operating system programs further include instructions for: receiving a user input indicative of a user identity; retrieving a user profile associated with the user identity; and configuring the device based on the user profile.

In some examples, the plurality of surgical software applications for processing surgical data are stored in one or more memories of the device. In some examples, the plurality of surgical software applications for processing surgical data are stored in one or more remote devices.

An exemplary method for providing a plurality of surgical software applications comprises: receiving, by one or more operating system programs of a device, one or more user inputs indicative of one or more surgical software applications of a plurality of surgical software applications of the device, wherein the plurality of surgical software applications include a plurality of sample input datasets corresponding to the plurality of surgical software applications; retrieving, by the one or more operating system programs of the device, one or more sample input datasets corresponding to the one or more surgical software applications from the plurality of sample input datasets; executing, by one or more processors of the device, based on the one or more sample input datasets, the one or more surgical software applications in a simulated environment; evaluating, by the one or more operating system programs of the device, the execution of the one or more surgical software applications; and outputting a recommendation related to the one or more surgical software applications.

In some examples, a sample input dataset of the plurality of sample input datasets comprises: image data, textual data, audio data, simulated user input data, or any combination thereof. In some examples, the sample input dataset of the plurality of sample input datasets comprises: surgical video data, medical record data, case file data, speech data, or any combination thereof.

In some examples, evaluating the execution of the one or more surgical software applications comprises: identifying a software crash event, a software hang event, a software lag event, a failure-to-launch event, an error, or any combination thereof. In some examples, evaluating the execution of the one or more surgical software applications comprises: evaluating usage of one or more memories of the device or the one or more processors of the device.

In some examples, the method further comprises receiving a user request to launch a surgical software application of the plurality of surgical software applications; retrieving one or more contract files associated with the surgical software application, wherein the one or more contract files comprise one or more parameters; comparing a current state of the device with the one or more parameters in the one or more contract files; and determining, based on the comparison between the current state of the device with the one or more parameters in the one or more contract files, whether to launch the surgical software application. In some examples, the method further comprises: in accordance with a determination to not launch the surgical software application, outputting a user notification.

In some examples, the one or more parameters in the one or more contract files comprise a first set of parameters specifying an amount of resource required to execute the surgical software application. In some examples, the first set of parameters is indicative of: a minimum memory requirement, a minimum core requirement, a minimum usage percentage, or any combination thereof. In some examples, the one or more parameters in the one or more contract files comprise a second set of parameters specifying one or more hardware components required to execute the surgical software application. In some examples, the one or more hardware components comprise: a central processing unit (CPU), a graphics processing unit (GPU), a capture card, an isolation port including a high-speed isolation port (which may be used in high-resolution camera systems, ultrasound devices, surgical tools, and the like), a USB port, or any combination thereof. In some examples, the one or more parameters in the one or more contract files comprise a third set of parameters specifying compatibility constraints of the surgical software application. In some examples, the third set of parameters is indicative of: one or more software environments that the surgical software application is compatible with. In some examples, the one or more parameters in the one or more contract files comprise a fourth set of parameters specifying a relative priority of the surgical software application. In some examples, the relative priority is determined based on a relative risk level associated with a surgical procedure. In some examples, the fourth set of parameters is indicative of: a priority value of the surgical software application and/or whether the surgical software application can run without a displayed graphical user interface.

In some examples, at least one surgical software application of the plurality of surgical software applications is subscription-based.

In some examples, the device further comprises a chassis and a temperature sensor inside the chassis, and the method further comprises: obtaining, via the temperature sensor, a temperature associated with the device; if the temperature exceeds a predefined temperature threshold, reducing an amount of power provided to the one or more processors.

In some examples, the method further comprises: if the temperature exceeds the predefined temperature threshold, terminating an active surgical software application being executed by the one or more processors. In some examples, the active surgical software application is selected based on a priority value associated with the active surgical software application and/or a last-used time associated with the active surgical software application. In some examples, the device comprises a fan, the method further comprising: if the temperature exceeds the predefined temperature threshold, increasing a speed of the fan. In some examples, the method further comprises receiving a user input indicative of a user identity; retrieving a user profile associated with the user identity; and configuring the device based on the user profile.

In some examples, the plurality of surgical software applications for processing surgical data are stored in one or more memories of the device. In some examples, the plurality of surgical software applications for processing surgical data are stored in one or more remote devices.

An exemplary non-transitory computer-readable storage medium stores one or more programs, the one or more programs comprising instructions, which when executed by one or more processors of an electronic device, cause the electronic device to perform any of methods described herein.

It will be appreciated that any of the variations, aspects, features and options described in view of the systems apply equally to the methods and vice versa. It will also be clear that any one or more of the above variations, aspects, features and options can be combined.

### BRIEF DESCRIPTION OF THE FIGURES

The invention will now be described, by way of example only, with reference to the accompanying drawings, in which:
FIG. 1 illustrates an exemplary operating room hub controller system, in accordance with some examples.
FIG. 2 illustrates a device for providing a plurality of surgical software applications, in accordance with some examples.
FIG. 3 illustrates an exemplary process for providing a plurality of surgical software applications, in accordance withs some examples.
FIG. 4 illustrates an exemplary method for performing pre-launch checks of a surgical software application, in accordance with some examples.
FIG. 5 illustrates an exemplary method for managing temperature of a device, in accordance with some examples.
FIGS. 6A-L illustrate exemplary user interfaces for a device configured to provide a plurality of surgical software applications, in accordance with some examples.
FIG. 6M illustrates an exemplary user interface for an external display, in accordance with some examples;
FIGS. 7A-D illustrate exemplary cooling ducts, in accordance with some examples.
FIG. 8 illustrates an exemplary electronic device, in accordance with some examples.

### DETAILED DESCRIPTION

Reference will now be made in detail to implementations and examples of various aspects and variations of systems and methods described herein. Although several exemplary variations of the systems and methods are described herein, other variations of the systems and methods may include aspects of the systems and methods described herein combined in any suitable manner having combinations of all or some of the aspects described.

Disclosed herein are exemplary devices, apparatuses, systems, methods, and non-transitory storage media for providing and testing a plurality of surgical software applications by utilizing sample input datasets. An exemplary device can comprise a plurality of surgical software applications for processing surgical data. Each surgical software application can include one or more sample input datasets that are configured to allow the surgical software to perform one or more of the software application's functionalities. The sample input dataset can comprise a variety of types of data, including image data, textual data, audio data, simulated user input data, or any combination thereof. The sample input dataset can be stored as a file or a collection of files (e.g., text file(s), audio file(s), or the like).

Using the sample input dataset(s), a user can test whether one or more surgical software applications can be safely and properly executed on the electronic device. For example, the device (e.g., one or more operating system programs of the device) can retrieve one or more sample input datasets corresponding to the one or more surgical software applications indicated by the user and execute, using the one or more sample input datasets, the one or more surgical software applications in a simulated environment. The device can then evaluate the execution of the one or more surgical software applications and output a recommendation related to the one or more surgical software applications. In some examples, the device outputs (e.g., displays) a recommendation to not execute the one or more surgical software applications on the device if an undesirable event has occurred during the execution of the one or more surgical software applications. In some examples, the device outputs a recommendation to not execute the one or more surgical software applications on the device if a parameter related to the execution (e.g., memory usage, processor usage, or the like) exceeds a predefined resource threshold. In some examples, the recommendation includes one or more reasons that the one or more surgical software applications cannot be run together or suggested actions to resolve the issue (e.g., acquiring certain software/hardware component, making certain software/hardware component of the device available, terminating one or more surgical software applications that are currently running, or the like).

Also disclosed herein are exemplary devices, apparatuses, systems, methods, and non-transitory storage media for performing pre-launch checks for a surgical software application. Upon receiving a user request to launch a surgical software application on a device, the device can first check the current state of the device and determine whether the current state of the device would allow the surgical software application to launch and operate properly. For example, each surgical software application of the device may include one or more corresponding contract files. The contract file(s) for each surgical software application can specify a plurality of parameters, such as: a first set of parameters specifying an amount of resources required to execute the surgical software application, a second set of parameters specifying one or more hardware components required to execute the surgical software application, a third set of parameters specifying one or more compatibility constraints of the surgical software application, a fourth set of parameters specifying a relative priority of the surgical software application, or the like. Based on the current state of the device and the parameters specified in the contract file(s), the electronic device may determine to not launch the surgical software application. The electronic device may alternatively determine to terminate or throttle a currently executing surgical software application in order to launch the new surgical software application (e.g., based on their priority values).

Also disclosed herein are exemplary devices, apparatuses, systems, methods, and non-transitory storage media for continuously monitoring a temperature associated with the device (e.g., via a temperature sensor inside the chassis of the device) and performing a series of mitigation actions if the temperature associated with the device exceeds a predefined temperature threshold to reduce the temperature of the device. The mitigation actions can include, for example, increasing the speed of a fan, enabling power saving modes on one or more processors, terminating one or more active surgical software applications (e.g., based on their priority values), or any combination thereof.

Also disclosed herein are cooling ducts for managing the temperature of an electronic device. The cooling ducts can include air vents to direct cooler air from outside the chassis of the device to processing units. The cooling ducts may be manufactured via 3D printing techniques. Manufacturing the cooling ducts via 3D printing can be more cost-efficient than molding, generate single pieces having complex geometries, and allow for quick updates to the designs due to changes to the processing units. Accordingly, the dimensions and shapes of the cooling ducts can be modified to be adapted to support different types of processing units to dissipate heat and reduce vibration, thus reducing the need for dedicated support brackets.

Examples of the present disclosure provide numerous technical advantages. They allow a user (e.g., a medical practitioner, a hospital administrator, a medical sales representative, a system administrator, a software developer, or the like) to test whether one or more surgical software applications can be properly and safely executed on an electronic device before surgical procedures. The tests can allow the user to launch surgical software applications in an informed manner to ensure surgical safety during surgical procedures. The tests can also help the user to evaluate a newly installed surgical software application to ensure that the electronic device can provide the resources to properly execute the surgical software application. Further, examples of the present disclosure can reduce the occurrence of undesirable events (e.g., a software crash event, a software hang event, a software lag event, a failure-to-launch event, an error, or the like) before and during surgical procedures by conducting pre-launch checks of a surgical software application and/or effectively managing the temperature of the device. Accordingly, examples of the present disclosure can result in a system that is more flexible, robust, efficient, and economical than conventional systems.

In the following description, it is to be understood that the singular forms "a," "an," and "the" used in the following description are intended to include the plural forms as well, unless the context clearly indicates otherwise. It is also to be understood that the term "and/or" as used herein refers to and encompasses any and all possible combinations of one or more of the associated listed items. It is further to be understood that the terms "includes," "including," "comprises," and/or "comprising," when used herein, specify the presence of stated features, integers, steps, operations, elements, components, and/or units but do not preclude the presence or addition of one or more other features, integers, steps, operations, elements, components, units, and/or groups thereof.

Certain aspects of the present disclosure include process steps and instructions described herein in the form of an algorithm. It should be noted that the process steps and instructions of the present disclosure could be embodied in software, firmware, or hardware and, when embodied in software, could be downloaded to reside on and be operated from different platforms used by a variety of operating systems. Unless specifically stated otherwise as apparent from the following discussion, it is appreciated that, throughout the description, discussions utilizing terms such as "processing," "computing," "calculating," "determining," "displaying," "generating," or the like refer to the action and processes of a computer system, or similar electronic computing device, that manipulates and transforms data represented as physical (electronic) quantities within the computer system memories or registers or other such information storage, transmission, or display devices.

The present disclosure in some examples also relates to a device for performing the operations herein. This device may be specially constructed for the required purposes, or it may comprise a general-purpose computer selectively activated or reconfigured by a computer program stored in the computer. Such a computer program may be stored in a non-transitory, computer readable storage medium, such as, but not limited to, any type of disk, including floppy disks, USB flash drives, external hard drives, optical disks, CD-ROMs, magnetic-optical disks, read-only memories (ROMs), random access memories (RAMs), EPROMs, EEPROMs, magnetic or optical cards, application specific integrated circuits (ASICs), or any type of media suitable for storing electronic instructions, and each coupled to a computer system bus. Furthermore, the computers referred to in the specification may include a single processor or may be architectures employing multiple processor designs for increased computing capability.

The methods, devices, and systems described herein are not inherently related to any particular computer or other apparatus. Various general-purpose systems may also be used with programs in accordance with the teachings herein, or it may prove convenient to construct a more specialized apparatus to perform the required method steps. The required structure for a variety of these systems will appear from the description below. In addition, the present invention is not described with reference to any particular programming language. It will be appreciated that a variety of programming languages may be used to implement the teachings of the present invention as described herein.

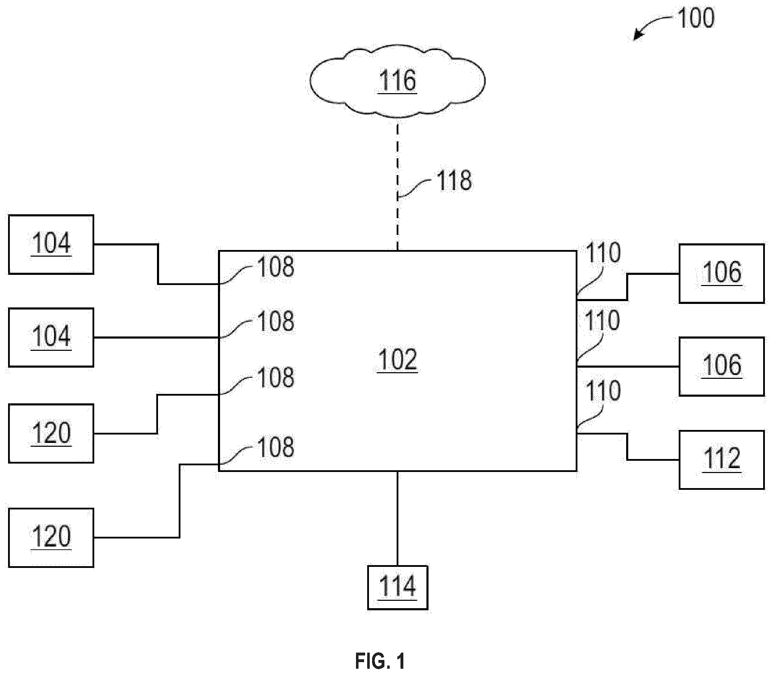

FIG. 1 illustrates a system 100 for generating and displaying medical imaging data during medical imaging sessions. System 100 includes a medical data processing device 102 that processes data received from one or more imaging modalities 104 to generate one or more display feeds for displaying enhanced medical imaging on one or more displays 106. The one or more imaging modalities 104 may generate image data associated with treatment of a patient. The image data can be images or videos generated during treatment of the patient in support of one or more medical procedures, such as video captured by an endoscopic camera during an endoscopic procedure on a patient. Examples of medical imaging modalities include, without limitation, endoscopic systems, open field imaging systems, x-ray systems such as intraoperative c-arm systems, computer tomography systems, ultrasound systems, magnetic resonance imaging systems, and nuclear medicine systems.

In some examples, the medical data processing device 102 may receive data from one or more non-imaging devices 120 that may be used in connection with (e.g., during) a medical imaging session and that may provide information that may be relevant for display during a medical imaging session. Non-limiting examples of non-imaging devices 120 include insufflators, arthroscopic pumps, illumination controllers, audio systems (e.g., microphone systems such as far-field microphone arrays, distributed microphones, and the like), and voice control systems.

The medical data processing device 102 may receive image data from the one or more imaging modalities 104 through one or more input ports 108. The medical data processing device 102 generates one or more display feeds using received imaging data and transmits the one or more display feeds to one or more displays 106 via one or more output ports 110. For example, the medical data processing device 102 may generate a display feed that includes enhanced imaging of tissue of a patient based on imaging generated by one or more imaging modalities 104, and the enhanced imaging may be displayed on one or more of the displays 106 to assist a user (e.g., a surgeon, a nurse, other medical personnel) during treatment of the patient. In some examples, one or more of the displays 106 may include an external touch screen that uses either one or more wires to connect to the medical data processing device 102 or connects wirelessly to the medical data processing device 102 to display information to the surgeon in the surgical environment. The medical data processing device 102 may also transmit display feeds to one or more recording devices 112 for recording enhanced imaging for later retrieval. Input ports 108 and output ports 110 may be any suitable types of data transmission ports, such as DP ports, DVI ports, HDMI ports, RS232 ports, IP ports, USB ports, or the like.

The medical data processing device 102 may be connected to one or more networks 116 via one or more network connections 118. The one or more networks 116 may be a local network such as a hospital information system or may be a wider network such as a wide area network or the internet. A network connection 118 can be a wired connection, such as an Ethernet connection, or a wireless network connection, such as a Wi-Fi connection. In some examples, the medical data processing device 102 may access the one or more networks 116 to retrieve configuration data stored at a network location for configuring the medical data processing device 102 for an imaging session, and/or may access the one or more networks to receive updated software and/or updated hardware files for processing imaging data. In some examples, one or more software applications can be received (e.g., downloaded) and installed onto the medical data processing device 102 and the one or more software applications can process medical data (e.g., medical image data) using the resources (e.g., memory, processor) of the medical data processing device 102. In some examples, the one or more software applications can access the resources of the medical data processing device 102 via an API structure.

One or more user interfaces 114 may be connected to the medical data processing device 102 for a user to provide input to the medical data processing device 102. The user may input data related to configuring the medical data processing device 102 for an imaging session. User input can include, for example, selection of a practitioner profile (e.g., a user profile as described herein) associated with an upcoming imaging session, selection of a type of imaging session or type of procedure to be performed during an imaging session, or any other relevant information. The one or more user interfaces 114 may include a tablet, a keyboard, a mouse, a voice control system, a keypad, a touchscreen, or any combination thereof. The user interface 114 may have a wired or wireless connection. The input may be provided locally or remotely such as off-site from the medical facility (e.g., by an administrator or third party).

### Testing of One or More Surgical Software Applications

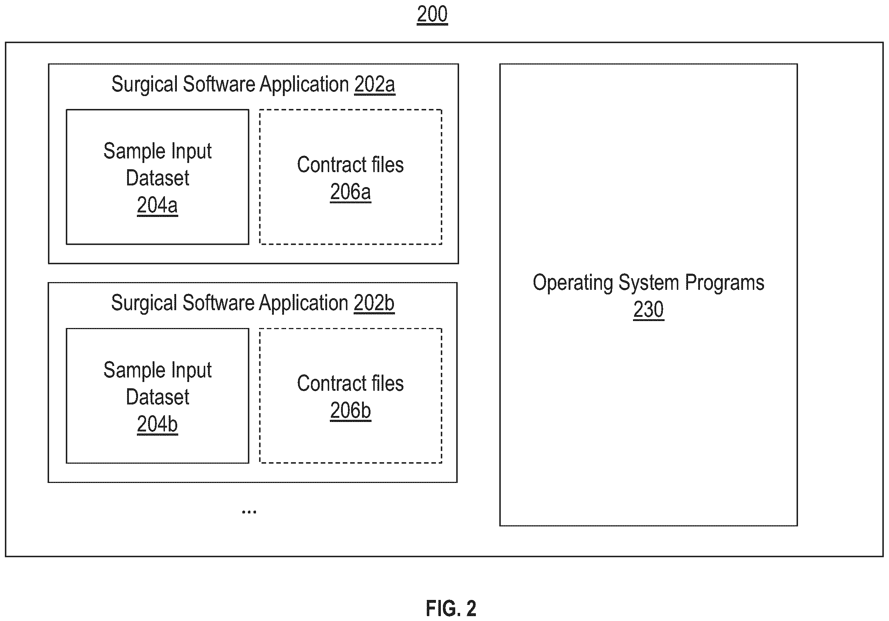

FIG. 2 illustrates a device 200 for providing a plurality of surgical software applications, in accordance with some examples. In some examples, the device 200 may be or include the medical data processing device 102 in FIG. 1 and can be configured to receive surgical data (e.g., surgical image data) and process the surgical data (e.g., to obtain enhanced surgical image data) using one or more surgical software applications, as described herein.

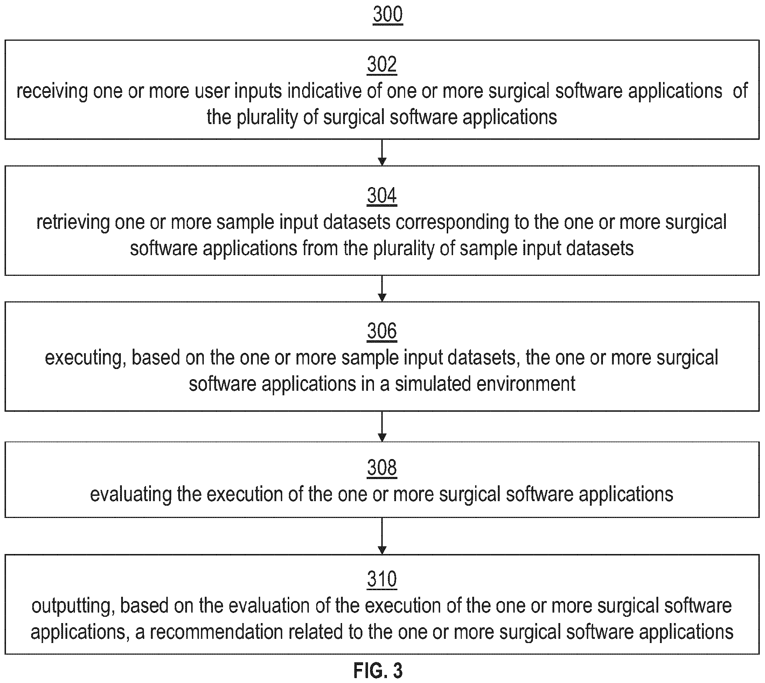

With reference to FIG. 2, the device 200 can comprise a plurality of surgical software applications for processing surgical data, such as surgical software application 202a and surgical software application 202b. Each surgical software application of the device 200 can include one or more corresponding sample input datasets, which can include one or more data packages or files. For example, with reference to FIG. 2, the surgical software application 202a includes a corresponding sample input dataset 204a and the surgical software application 202b includes a corresponding sample input dataset 204b. The sample input datasets can be used to determine if one or more surgical software applications can be safely and properly executed on the device 200, as described herein in detail with reference to FIG. 3.

A sample input dataset of a surgical software application can include data that can be used by the surgical software application to perform one or more functionalities of the surgical software application. The sample input dataset can be stored as a file or a collection of files (e.g., text file(s), audio file(s), or the like). The sample input dataset can comprise a variety of types of data, including image data, textual data, audio data, simulated user input data, or any combination thereof. The image data may include one or more images of a patient's anatomy (e.g., surgical video data), one or more images of the surgical environment (e.g., images of the operating room), one or more images of objects and people in the surgical environment, or any combination thereof. The textual data may include medical record data, case file data, readings from medical devices and sensors (e.g., pressure measurements and flow rate measurements from an endoscopy pump), or any combination thereof. The audio data may include speech data (e.g., voice commands), sounds associated with a surgical procedure or surgical environment, or any combination thereof. In some aspects, the audio data may be or include prerecorded voice commands and/or sounds associated with a surgical procedure or a surgical environment, which are encoded within digital audio files. Examples of audio files used to store audio data may include AIF, AIFF, WAV, MP3, and the like. In some aspects, the audio data may be processed by a dedicated audio application installed on, or otherwise accessible to, the medical data processing device 200. In some examples, one or more microphones (e.g., far-field microphone array, distributed microphones, and the like) may be communicably coupled to the medical data processing device 200 (e.g., via one or more ports of the medical data processing device 200) such that the dedicated audio application may bypass any signals captured by the one or more microphones to process the prerecorded audio data when executing a sample input dataset in the simulated environment. The simulated user input data may include a user's input for the surgical software application (e.g., selection of a functionality or setting, input of a value).

The sample input dataset can include data that would require a relatively high resource utilization (e.g., processing power, battery power, memory usage, or the like) to be processed by the surgical software application. For example, a surgical software application may provide a plurality of features and the image enhancement feature may require the highest resource utilization among the plurality of features. Accordingly, the sample input dataset may include surgical images to be enhanced by the surgical software application. As another example, the sample input dataset may include image data having the highest resolution that the surgical software application is designed to handle. As another example, the sample input dataset may include video data having the highest frame rate that the surgical software application is designed to handle.

In some examples, a surgical software application (not depicted in FIG. 2) may include multiple sample input datasets that would impose different levels of computational burden on the device. For example, a surgical software application may include a first sample input dataset that would impose a higher level of computational burden because it includes video data with a higher frame rate, image data with a higher resolution, user inputs associated with features having a higher utilization of the user interfaces, etc., and a second sample input dataset that would impose a lower level of computational burden because it includes video data with a lower frame rate, image data with a lower resolution, user inputs associated with features having a lower utilization of the user interfaces, etc.

Different surgical software applications may include different types of sample input datasets. For example, the sample input dataset for a surgical software application configured to identify lymph nodes in an image may include a plurality of images of lymph nodes. In contrast, the sample input dataset for a surgical software application configured to identify blood vessels in an image may include a plurality of images of blood vessels.

Further, a sample input dataset for a surgical software application may comprise multiple types of data. As described herein, a sample input dataset can be stored as a file or a collection of files (e.g., text file(s), audio file(s), or the like). For example, a surgical software application may provide a plurality of functionalities including understanding voice commands and enhancing surgical images. Accordingly, the sample input dataset for the surgical software application may include both audio data (e.g., audio files that include voice commands to be processed) and surgical images to be enhanced.

Further, a sample input dataset may specify a sequence of sample user inputs simulating a typical workflow. For example, the sample input dataset may include an audio file, data indicating a user selection, and a video, and further specify that the surgical software application is to first process the audio file (e.g., a speech input to launch a menu), then process the user selection data (e.g., a user selection of a feature in the menu), and then process the video in accordance with the selected feature (e.g., via a video processing pipeline to extract images and process the images based on the selected feature). As another example, the sample input dataset may include data indicating one or more software or hardware button presses, the associated timestamps for the button presses, and sample data captured from other devices (e.g., anesthesiology machines, electrocardiogram devices, infusion pumps, and the like) and further specify that the surgical software application is to first process the one or more button presses and the associated timestamps to identify selected feature(s) and then process the sample data from other devices in accordance with the selected feature.

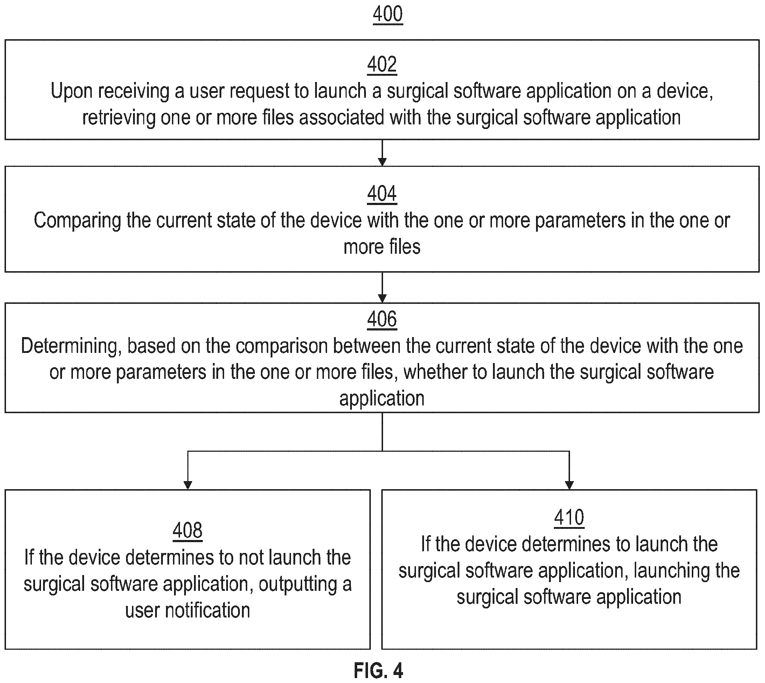

With reference to FIG. 2, each surgical software application of the device 200 may include one or more corresponding contract files. For example, with reference to FIG. 2, the surgical software application 202a includes a corresponding set of contract files 206a and the surgical software application 202b includes a corresponding set of contract files 206b. The contract file(s) for each surgical software application can include a plurality of parameters that can be used to determine, upon a user request to launch the surgical software application on the device 200, whether the current state of the device 200 would allow the surgical software application to be safely and properly launched and executed, as described in detail with reference to FIG. 4.

In some examples, the one or more parameters in the one or more contract files comprise a first set of parameters specifying an amount of resources required to execute the surgical software application. For example, the first set of parameters can be indicative of: a minimum memory requirement, a minimum core requirement, a minimum usage percentage, or any combination thereof. The first set of parameters can be related to a central processing unit (CPU), a graphics processing unit (GPU), or both. Thus, the device 200 can compare the first set of parameters with the current state of the device 200 (e.g., memory available, processing power available at the CPU, processing power available at the GPU, or the like) to determine if the device 200 currently can provide the resources required to execute the surgical software application. If the device 200 cannot provide the resources required to execute the surgical software application, the device 200 can determine to not launch the surgical software application.

In some examples, the one or more parameters in the one or more contract files comprise a second set of parameters specifying one or more hardware components required to execute the surgical software application. For example, the one or more hardware components comprise: a CPU, a GPU, a capture card, communication hardware (e.g., a communication bus, a network hardware device), an isolation port, a USB port (e.g., USB-C port), an external device (e.g., an external medical device), or any combination thereof. Thus, the device 200 can compare the second set of parameters with the current state of the device 200 (e.g., hardware components available) to determine if the device 200 currently can provide the one or more hardware components required to execute the surgical software application. If the device 200 cannot provide the one or more hardware components required to execute the surgical software application, the device 200 can determine to not launch the surgical software application.

In some examples, the one or more parameters in the one or more contract files comprise a third set of parameters specifying one or more compatibility constraints of the surgical software application. For example, the third set of parameters can be indicative of: one or more software environments (e.g., a version of an operating system) that the surgical software application is compatible with. Thus, the device 200 can compare the third set of parameters with the current state of the device 200 to determine if the device 200 (e.g., the operating system installed on the device 200) is compatible with the surgical software application. If the device 200 is not compatible with the surgical software application, the device 200 can determine to not launch the surgical software application.

In some examples, the one or more parameters in the one or more contract files comprise a fourth set of parameters specifying a relative priority of the surgical software application. In some examples, the fourth set of parameters is indicative of: a priority value of the surgical software application. For example, a surgical software application that may be run in the background (e.g., without a displayed graphical user interface) to collect data may be associated with a lower priority value. In contrast, a surgical software application that provides a graphical user interface that offers visual guidance to a surgeon may be associated with a higher priority value. In some examples, the relative priority can be based on a relative risk level associated with a surgical procedure or risk to patient safety. For example, a surgical software application associated with a high-risk surgical procedure may be associated with a higher priority value.

Thus, the device 200 can compare the fourth set of parameters with the current state of the device 200 (e.g., the priority values of the currently running software applications). If the surgical software application cannot be launched without terminating or throttling one or more currently running software applications with lower priority values, the device 200 can determine to not launch the surgical software application. The fourth set of parameters can be used to decide if performance of a currently executing software application needs to be throttled (e.g., let the currently executing software application hang or run slower) so that a new software application with a higher priority value can be launched.

The one or more contract files associated with a surgical software application may include one or more parameters and/or instructions defining a preinstallation process that may be performed by device 200 prior to installation of the given surgical software application. The preinstallation process may prepare the device for installation of the surgical software application. The preinstallation process may include uninstalling a previous version of the surgical software application. The preinstallation process may include deleting and/or modifying files and/or data associated with a previous version of the surgical software application. For example, modifying files and/or data associated with a previous version of the surgical software application may include reformatting data (such as, image data, textual data, audio data, simulated user input data, or any combination thereof) stored on the device and used with the previous version of the surgical software application. The data may be reformatted according to the requirements of a newer version of the surgical software application to be installed. The one or more contract files associated with a surgical software application may include one or more parameters and/or instructions defining a postinstallation process that may be performed by device 200 after installation of the given surgical software application. The postinstallation process may include, for example, removing installation files and/or data.

In some examples, at least one surgical software application of the plurality of surgical software applications on the device 200 can be subscription-based. The plurality of surgical software applications for processing surgical data can be stored in one or more memories of the device and/or stored in one or more remote devices (which can be accessed via a network).

Further with reference to FIG. 2, the device 200 can comprise one or more operating system programs 230 for testing and providing the plurality of surgical software applications, as described below with reference to FIG. 3. The device 200 can further comprise one or more processors (not depicted) for executing the plurality of surgical software applications and the one or more operating system programs.

FIG. 3 illustrates an exemplary process 300 for providing a plurality of surgical software applications, in accordance with some examples. Process 300 may be performed by one or more programs of a device (e.g., device 200 in FIG. 2, medical data processing device 102 in FIG. 1, or the like) to test surgical software applications on the device. For example, with reference to the depicted example in FIG. 2, the process 300 may be performed by the one or more operating system programs 230 of the device 200 to test one or more surgical software applications (e.g., 202a, 202b) on the device 200. In some examples, process 300 is performed using a client-server system, and the blocks of process 300 are divided up in any manner between the server and one or more client devices. In some examples, process 300 is performed using only a client device or only multiple client devices. In process 300, some blocks are, optionally, combined, the order of some blocks is, optionally, changed, and some blocks are, optionally, omitted. In some examples, additional steps may be performed in combination with the process 300. Accordingly, the operations as illustrated (and described in greater detail below) are exemplary by nature and, as such, should not be viewed as limiting.

At block 302, an exemplary device (e.g., device 200 in FIG. 2) receives one or more user inputs indicative of one or more surgical software applications of the plurality of surgical software applications. The user providing the user inputs may be any user that wishes to determine if one or more surgical software applications can be properly executed on the device. In some examples, the user may include a medical practitioner (e.g., a surgeon, a nurse, a surgical staff member), a hospital administrator, a medical sales representative, a system administrator, a software developer, etc. In some examples, the device can include a display for providing a user interface that shows the plurality of surgical software applications available to the user on the device and allows the user to select the one or more surgical software applications from the user interface.

In some examples, the one or more user inputs indicate a single surgical software application. This may occur if the user would like to test if the device is equipped with the resources needed (e.g., processors, memory) to properly execute the single surgical software application. In some examples, the device may be configured to only execute a single surgical software application at a time. In some examples, the one or more user inputs indicate multiple surgical software applications. This may occur if the user would like to test if the device is equipped with the resources needed to properly execute the multiple surgical software applications simultaneously.

At block 304, the device retrieves one or more sample input datasets corresponding to the one or more surgical software applications from the plurality of sample input datasets. For example, with reference to FIG. 2, if the device 200 receives a user input selecting the surgical software application 202a and the surgical software application 202b for testing, the device can retrieve the sample input dataset 204a (corresponding to the surgical software application 202a) and the sample input dataset 204b (corresponding to the surgical software application 202b) accordingly.

A sample input dataset of a surgical software application can include data that can be used by the surgical software application to perform one or more functionalities of the surgical software application. The sample input dataset can be stored as a file or a collection of files (e.g., text file(s), audio file(s), or the like). The sample input dataset can comprise a variety of types of data, including image data, textual data, audio data, simulated user input data, or any combination thereof. The image data may include one or more images of a patient's anatomy (e.g., surgical video data), one or more images of the surgical environment (e.g., images of the operating room), one or more images of objects and people in the surgical environment, or any combination thereof. The textual data may include medical record data, case file data, readings from medical devices and sensors (e.g., pressure measurements and flow rate measurements from an endoscopy pump), or any combination thereof. The audio data may include speech data (e.g., voice commands), sounds associated with a surgical procedure or surgical environment, or any combination thereof. In some aspects, the audio data may be or include prerecorded voice commands and/or sounds associated with a surgical procedure or a surgical environment, which are encoded within digital audio files. Examples of audio files used to store audio data may include AIF, AIFF, WAV, MP3, and the like. In some aspects, the audio data may be processed by a dedicated audio application installed on, or otherwise accessible to, the medical data processing device 200. In some examples, one or more microphones (e.g., far-field microphone array, distributed microphones, and the like) may be communicably coupled to the medical data processing device 200 (e.g., via one or more ports of the medical data processing device 200) such that the dedicated audio application may bypass any signals captured by the one or more microphones to process the prerecorded audio data when executing a sample input dataset in the simulated environment. The simulated user input data may include a user's input for the surgical software application (e.g., selection of a functionality or setting, input of a value).

The sample input dataset can include data that would require a relatively high resource utilization (e.g., processing power, battery power, memory usage, or the like) to be processed by the surgical software application. For example, a surgical software application may provide a plurality of features and the image enhancement feature may require the highest resource utilization among the plurality of features. Accordingly, the sample input dataset may include surgical images to be enhanced by the surgical software application. As another example, the sample input dataset may include image data having the highest resolution that the surgical software application is designed to handle. As another example, the sample input dataset may include video data having the highest frame rate that the surgical software application is designed to handle.

In some examples, a surgical software application (not depicted in FIG. 2) may include multiple sample input datasets that would impose different levels of computational burden on the device. For example, a surgical software application may include a first sample input dataset that would impose a higher level of computational burden because it includes video data with a higher frame rate, image data with a higher resolution, user inputs associated with features having a higher utilization of the user interfaces, etc., and a second sample input dataset that would impose a lower level of computational burden (in relation to the first sample input dataset) because it includes video data with a lower frame rate, image data with a lower resolution, user inputs associated with features having a lower utilization of the user interfaces, etc.

Different surgical software applications may include different types of sample input datasets. For example, the sample input dataset for a surgical software application configured to identify lymph nodes in an image may include a plurality of images of lymph nodes. In contrast, the sample input dataset for a surgical software application configured to identify blood vessels in an image may include a plurality of images of blood vessels.

Further, a sample input dataset for a surgical software application may comprise multiple types of data. For example, a surgical software application may provide a plurality of functionalities including understanding voice commands and enhancing surgical images. Accordingly, the sample input dataset for the surgical software application may include both audio data (e.g., voice commands to be processed) and surgical images to be enhanced.

Further, a sample input dataset may specify a sequence of sample user inputs simulating a typical workflow. For example, the sample input dataset may include an audio file, data indicating a user selection, and a video, and further specify that the surgical software application is to first process the audio file (e.g., a speech input to launch a menu), then process the user selection data (e.g., a user selection of a feature in the menu), and then process the video in accordance with the selected feature (e.g., via a video processing pipeline to extract images and processing the images based on the selected feature). As another example, the sample input dataset may include data indicating one or more software or hardware button presses, the associated timestamps for the button presses, and sample data captured from other devices (e.g., anesthesiology machines, electrocardiogram devices, infusion pumps, and the like) and further specify that the surgical software application is to first process the one or more button presses and the associated timestamps to identify selected feature(s) and then process the sample data from other devices in accordance with the selected feature.

At block 306, the device executes, based on the one or more sample input datasets, the one or more surgical software applications. In some examples, the device executes the one or more surgical software applications in a simulated environment. Execution of the one or more surgical software applications in the simulated environment can refer to a simulated run or execution of the one or more surgical software applications using the existing resources of the device (e.g., prior to a surgical procedure). Examples of events that may prompt this early, simulated run using the predefined sample input dataset(s) may include the addition of a new surgical software application to the device, the addition of a new connected device/console, the update of software and/or firmware, and the like.

At block 308, the device evaluates the execution of the one or more surgical software applications. In some examples, the device can evaluate the execution by detecting if an undesirable event has occurred. The undesirable event may include any event where a software or hardware component of the device fails to operate properly, such as software crash event, a software hang event, a software lag event, a failure-to-launch event, an error (e.g., operating with a lower frame rate), network issues, or any combination thereof. For example, if the surgical software application requires a hardware component or a software component (e.g., an older version of an operating system) that the device does not provide, the surgical software application may fail to launch. As another example, if multiple surgical software applications are executed to run simultaneously and the collective resources utilized by the multiple surgical software applications (e.g., memory usage, processor usage, or the like) exceeds what the device can provide, a software crash event, a software hang event, or a software lag event may occur. As another example, if multiple surgical software applications are incompatible with each other (e.g., they require the same resources such as a capture card or an isolation port) if run simultaneously, a failure-to-launch or a software crash event may occur. As another example, if multiple surgical software applications altogether require more collective network bandwidth than what the device can provide, network issues or software crash/hang/lag events may occur. In some examples, the device can evaluate one or more parameters related to the execution (e.g., memory usage, processor usage, or the like) and compare the parameters against one or more predefined resource thresholds to determine if any of the predefined resource thresholds is exceeded. For example, a predefined resource threshold for a surgical software application may specify that the surgical software application should not consume more than 10% of the CPU, more than 1000Mb of memory usage, and/or more than 4 CUDA Cores of the GPU, when the surgical software application is run by itself. As another example, a predefined resource threshold for the entire device may specify that the surgical software applications should not collectively consume more than a certain percentage of the CPU. Additional examples of the parameters are provided with reference to FIG. 4 described herein.

At block 310, the device outputs, based on the evaluation of the execution of the one or more surgical software applications, a recommendation related to the one or more surgical software applications. In some examples, the device outputs (e.g., displays) a recommendation to not execute the one or more surgical software applications on the device if an undesirable event has occurred during the execution of the one or more surgical software applications in block 306. In some examples, the device outputs a recommendation to not execute the one or more surgical software applications on the device if a parameter related to the execution (e.g., memory usage, processor usage, or the like) exceeds a predefined resource threshold. In some examples, the recommendation includes one or more reasons that the one or more surgical software applications cannot be run together or suggested actions to resolve the issue (e.g., acquiring certain software/hardware component, making certain software/hardware component of the device is available, terminating one or more surgical software applications that are currently running, or the like).

Optionally, in addition to outputting a recommendation, or instead of outputting the recommendation, the device may determine whether or not to launch the one or more surgical software applications. The device may determine not to launch the one or more surgical software applications on the device if an undesirable event has occurred during the execution of the one or more surgical software applications in block 306. The device may determine not to launch the one or more surgical software applications on the device if a parameter related to the execution (e.g., memory usage, processor usage, or the like) exceeds a predefined resource threshold. For instance, if the surgical software application cannot be launched without terminating or throttling one or more currently running software applications with lower priority values, the device can determine to not launch the surgical software application. The device may provide a notification to the user to terminate or throttle one or more currently running software applications with lower priority values. The device may provide a notification to the user that the device will automatically terminate or throttle one or more currently running software applications with lower priority values unless such automatic terminating or throttling is aborted by the user. Alternatively, the device may automatically terminate or throttle one or more currently running software applications with lower priority values, without user confirmation.

In accordance with determining to launch the one or more surgical software applications, the device may proceed with launching the one or more surgical software applications. Proceeding with launching the one or more surgical software applications may include a pre-launch check of one or more surgical software applications in accordance with the principles described below with respect to method 400 of FIG. 4. Alternatively, the device may launch the one or more surgical software applications without a pre-launch check. The device may prompt a user to confirm whether to proceed with launching of the one or more surgical software applications, or the device may proceed with launching automatically, without user confirmation.

In accordance with determining not to launch, the device may not launch the one or more surgical software applications. The device may provide one or more notifications associated with the one or more surgical software applications not being launched, which may include a notification as discussed above with respect to step 310. Optionally, the device may prevent launch of the one or more surgical software applications such that a user cannot launch the one or more surgical software applications. Optionally, the device may prevent launch of the one or more surgical software applications until a notification has been acknowledged by a user. For example, the device may display a notification that the one or more surgical software applications failed the evaluation and will not be launched unless a user acknowledges and clears the notification.

### Pre-launch Check of a Surgical Software Application

Upon receiving a user request to launch a surgical software application on a device, the device (e.g., device 200) can first check the current state of the device and only launch the surgical software application upon determining that the current state of the device would allow the surgical software application to launch and operate properly. FIG. 4 illustrates an exemplary method 400 for performing pre-launch checks of a surgical software application, in accordance with some examples. For example, with reference to the depicted example in FIG. 2, the process 400 may be performed by the one or more operating system programs 230 of the device 200 in FIG. 2 to determine if a surgical software application (e.g., 202a, 202b) can be launched on the device based on a current state of the device 200.

In some examples, process 400 is performed using a client-server system, and the blocks of process 400 are divided up in any manner between the server and one or more client devices. In some examples, process 400 is performed using only a client device or only multiple client devices. In process 400, some blocks are, optionally, combined, the order of some blocks is, optionally, changed, and some blocks are, optionally, omitted. In some examples, additional steps may be performed in combination with the process 400. Accordingly, the operations as illustrated (and described in greater detail below) are exemplary by nature and, as such, should not be viewed as limiting.

At block 402, upon receiving a user request to launch a surgical software application on the device, the device can retrieve one or more contract files associated with the surgical software application. At block 404, the device can then compare the current state of the device with the one or more parameters in the one or more contract files. At block 406, the device can determine, based on the comparison between the current state of the device with the one or more parameters in the one or more contract files, whether to launch the surgical software application.

In some examples, the one or more parameters in the one or more contract files comprise a first set of parameters specifying an amount of resources required to execute the surgical software application. For example, the first set of parameters can be indicative of: a minimum memory requirement, a minimum core requirement, a minimum usage percentage, or any combination thereof. The first set of parameters can be related to a central processing unit (CPU), a graphics processing unit (GPU), or both. Thus, the device can compare the first set of parameters with the current state of the device (e.g., memory available, processing power available at the CPU, processing power available at the GPU, or the like) to determine if the device currently can provide the resources required to execute the surgical software application. If the device cannot provide the resources required to execute the surgical software application, the device can determine to not launch the surgical software application.

In some examples, the one or more parameters in the one or more contract files comprise a second set of parameters specifying one or more hardware components required to execute the surgical software application. For example, the one or more hardware components comprise: a CPU, a GPU, a capture card, communication hardware (e.g., a communication bus, a network hardware device), an isolation port, a USB port (e.g., USB-C port), an external device (e.g., an external medical device), or any combination thereof. Thus, the device can compare the second set of parameters with the current state of the device (e.g., hardware components available) to determine if the device currently can provide the one or more hardware components required to execute the surgical software application. If the device cannot provide the one or more hardware components required to execute the surgical software application, the device can determine to not launch the surgical software application.

In some examples, the one or more parameters in the one or more contract files comprise a third set of parameters specifying one or more compatibility constraints of the surgical software application. For example, the third set of parameters can be indicative of: one or more software environments (e.g., a version of an operating system) that the surgical software application is compatible with. Thus, the device can compare the third set of parameters with the current state of the device to determine if the device (e.g., the operating system installed on the device) is compatible with the surgical software application. If the device is not compatible with the surgical software application, the device can determine to not launch the surgical software application.

In some examples, the one or more parameters in the one or more contract files comprise a fourth set of parameters specifying a relative priority of the surgical software application. In some examples, the fourth set of parameters is indicative of: a priority value of the surgical software application. For example, a surgical software application that may be run in the background (e.g., without a displayed graphical user interface) to collect data may be associated with a lower priority value. In contrast, a surgical software application that provides a graphical user interface that offers visual guidance to a surgeon may be associated with a higher priority value. In some examples, the relative priority can be based on a relative risk level associated with a surgical procedure or risk to patient safety. For example, a surgical software application associated with a high-risk surgical procedure may be associated with a higher priority value.

Thus, the device can compare the fourth set of parameters with the current state of the device (e.g., the priority values of the currently running software applications). If the surgical software application cannot be launched without terminating or throttling one or more currently running software applications with lower priority values, the device can determine to not launch the surgical software application. The fourth set of parameters can be used to decide if performance of a currently executing software application needs to be throttled (e.g., let the currently executing software application hang or run slower) so that a new software application with a higher priority value can be launched.

Thus, the device can compare any one or more, such as all, of the first, second, third and fourth sets of parameters with the current state of the device for determining whether or not to launch the surgical software application

At block 408, if the device determines to not launch the surgical software application, the device may output (e.g., display) a user notification. The user notification may indicate that the surgical software application cannot be launched, one or more reasons (e.g., lack of resources, lack of required hardware components, incompatibility with the current operating system, higher-priority applications are running), and/or one or more suggested user actions to allow the surgical software application to be launched (e.g., plugging in a missing hardware component, terminating a currently running software application). Optionally, the device may enable the user to clear the notification and proceed with launching with or without the user taking one or more suggested actions.

At block 410, if the device determines to launch the surgical software application, the device may proceed to allocate resources and launch the surgical software application. In some examples, the device can start to monitor the resources. In some examples, the system may monitor GPU utilization (e.g., 0-100%), GPU memory utilization, CPU memory utilization, the utilization of available input connections such as zero latency video ports and USB ports (which may be connected to touchscreens, printers, or other external USB devices), or any combination thereof. In some examples, the system may monitor the wireless bandwidth used for Wi-Fi hotspot (which may be used by extended reality devices), utilization of hard disk space, and/or utilization of capture card for obtaining C-arm images.

### Temperature Management

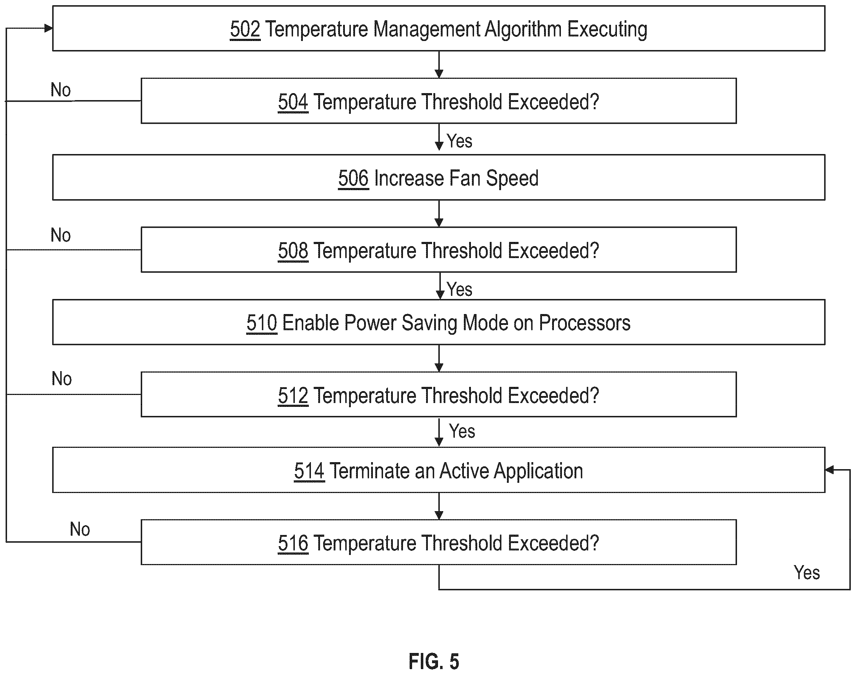

An exemplary device (e.g., device 200 in FIG. 2) can continuously monitor a temperature associated with the device (e.g., via a temperature sensor inside the chassis of the device) and take a series of mitigation actions if the monitored temperature associated with the device exceeds a predefined temperature threshold. FIG. 5 illustrates an exemplary method 500 for managing temperature of a device, in accordance with some examples. For example, with reference to the depicted example in FIG. 2, the process 500 may be performed by the one or more operating system programs 230 of the device 200 in FIG. 2 to manage power on the device 200. In some examples, process 500 is performed using a client-server system, and the blocks of process 500 are divided up in any manner between the server and one or more client devices. In some examples, process 500 is performed using only a client device or only multiple client devices. In process 500, some blocks are, optionally, combined, the order of some blocks is, optionally, changed, and some blocks are, optionally, omitted. In some examples, additional steps may be performed in combination with the process 500. Accordingly, the operations as illustrated (and described in greater detail below) are exemplary by nature and, as such, should not be viewed as limiting.

At block 502, the temperature management algorithm starts execution. In some examples, block 502 may be triggered upon a user input (e.g., a user input to request execution of the temperature management algorithm). In some examples, block 502 can be triggered when the device starts up. In some examples, block 502 can be triggered when one or more conditions are met (e.g., when a new surgical software application is launched on the device, when the device is connected to a new peripheral device).

At block 504, the device determines if the temperature associated with the device exceeds a predefined temperature threshold. The temperature associated with the device can be obtained, for example, via a temperature sensor inside a chassis of the device. In accordance with a determination that the predefined temperature threshold is not exceeded, the method returns to block 502 such that the temperature associated with the device can be continually monitored. On the other hand, in accordance with a determination that the predefined temperature threshold is exceeded, the device can perform a series of mitigation actions per the order specified by the method 500. It should be appreciated that the order specified by the method 500 in FIG. 5 is merely exemplary and that the mitigation actions may be performed in a different order.

At block 506, in accordance with a determination that the predefined temperature threshold is exceeded, the device performs a first mitigation action. In the depicted example, the first mitigation action includes increasing the speed of a fan of the device. In some examples, the device may progressively increase the speed of the fan until a maximum speed is reached.

At block 508, the device determines if the temperature associated with the device still exceeds the predefined temperature threshold. The temperature associated with the device can be obtained, for example, via the temperature sensor inside the chassis of the device after the first mitigation action is performed. In accordance with a determination that the predefined temperature threshold is not exceeded, the method returns to block 502 such that the temperature associated with the device can be continually monitored.

At block 510, in accordance with a determination that the predefined temperature threshold is still exceeded, the device performs a second mitigation action. In the depicted example, the second mitigation action includes enabling power saving mode on one or more processors (e.g., CPU, GPU, or the like) of the device. For example, the device can reduce the maximum amount of power that a processor is allowed to draw, reduce a clock speed associated with the processor, limit execution to less processing cores on the processor, and the like.

At block 512, the device determines if the temperature associated with the device still exceeds the predefined temperature threshold. The temperature associated with the device can be obtained, for example, via the temperature sensor inside the chassis of the device after the second mitigation action is performed. In accordance with a determination that the predefined temperature threshold is not exceeded, the method returns to block 502 such that the temperature associated with the device can be continually monitored.

At block 514, in accordance with a determination that the predefined temperature threshold is still exceeded, the device performs a third mitigation action. In the depicted example, the third mitigation action includes terminating an active surgical software application on the device. In some examples, the device can compare the priority values of the currently executing surgical software applications (e.g., as specified in the contract files described herein) and terminate the surgical software application with the lowest priority value. In some examples, the device can terminate the surgical software application with the least recent last-use time.

At block 516, the device determines if the temperature associated with the device still exceeds the predefined temperature threshold. The temperature associated with the device can be obtained, for example, via the temperature sensor inside the chassis of the device after the third mitigation action is performed. In accordance with a determination that the predefined temperature threshold is still exceeded, the device may return to block 514 to terminate another surgical software application (e.g., with the lowest priority value or with the least recent last-use time). In accordance with a determination that the predefined temperature threshold is not exceeded, the method returns to block 502 such that the temperature associated with the device can be continually monitored and a series of mitigation actions may be taken if the temperature associated with the device exceeds the predefined temperature threshold per the order specified by the method 500.

### Loading Software Applications on New Device

An exemplary device (e.g., device 200) can be automatically configured and set up based on a user profile. For example, if a user acquires a new device or acquires a temporary device while their existing device is being serviced, the new device can be automatically configured such that the user's software applications, files, layouts, and settings from a previous device are automatically loaded onto the new device. This way, the user does not need to manually acquire (e.g., download) their previous applications to the new device and manually configure the new device. In some examples, the device (e.g., one or more operating system programs of the device) can receive a user input indicative of a user identity (e.g., by user inputs of credentials). The device can then retrieve a user profile associated with the user identity. The user profile can include information related to the user's software applications, settings, files, layouts, etc. The user profile may be retrieved from a remote device via a network (e.g., from the cloud) or from a local storage device. The device can then configure the device based on the user profile. Configurating the device can include acquiring (e.g., downloading) applications and installing them on the device, acquiring data files and storing them on the device, and configuring the device according to the settings in the user profile.

### Exemplary Graphical User Interfaces

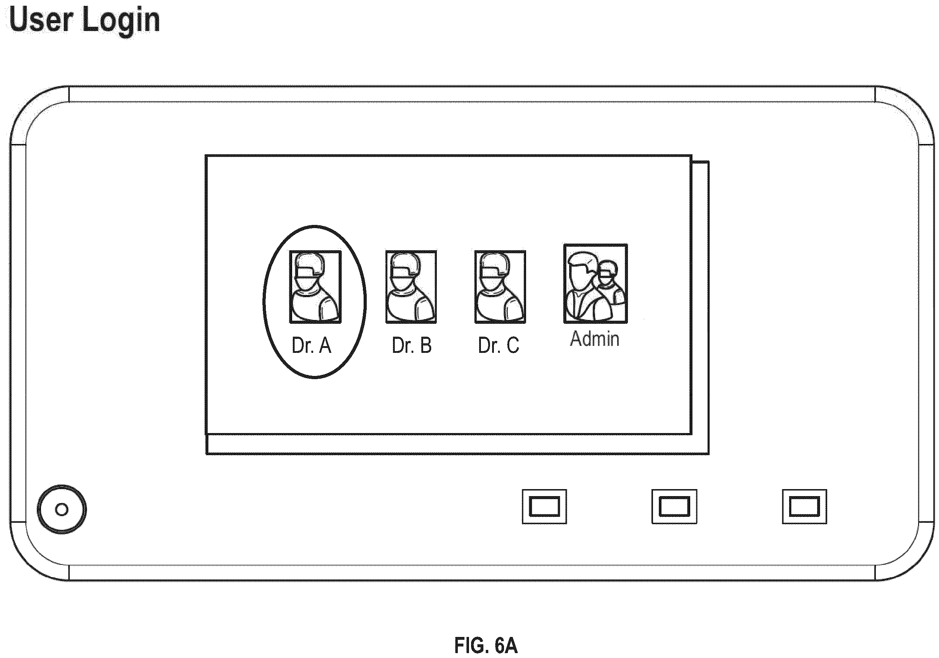

FIGS. 6A-K illustrate exemplary user interfaces for a device configured to provide a plurality of surgical software applications, in accordance with some examples. The device may be device 200 in FIG. 2, medical data processing device 102 in FIG. 1, or the like. The exemplary user interfaces as described herein may receive user inputs via any input device (e.g., touchscreen, mouse, keyboard, trackpad, joystick, microphone, gesture recognition devices, and the like).

FIG. 6A illustrates an exemplary user interface for allowing a user to log into the device. As shown, the device can provide a plurality of user accounts for selection. Once a user (e.g., a surgeon, an administrator) selects a user account from the plurality of user accounts, the device can automatically log into the user's selected account (e.g., based on previously provided user credentials) or display additional graphical user interfaces to allow the user to provide his or her credentials to log into the device. In the depicted example in FIG. 6A, the user selects the user account corresponding to "Dr. A."

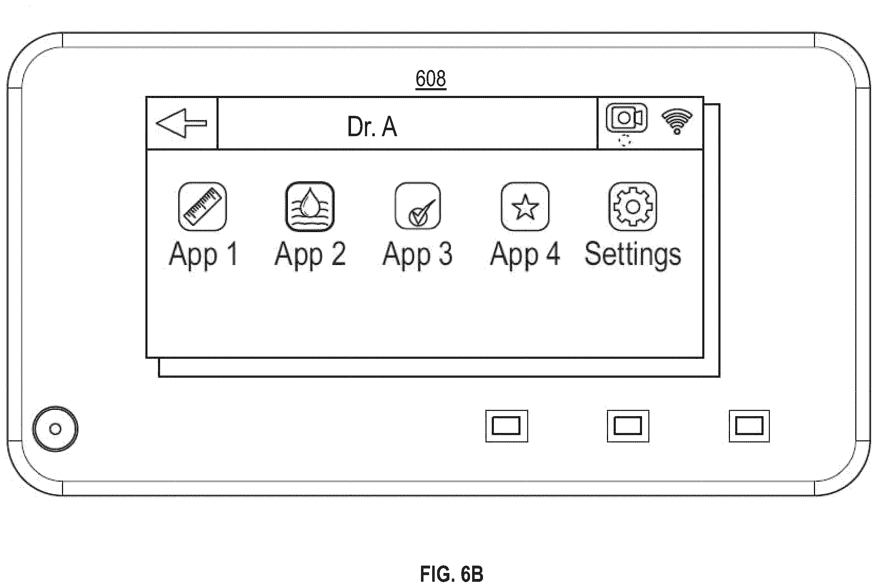

Once the user logs into the device, the device can display a user-specific home screen as shown in FIG. 6B. As shown, the user-specific home screen can include a plurality of surgical software applications that the user may access. In the depicted example in FIG. 6B, the device displays a home screen 608 specifically for Dr. A, which provides a plurality of icons corresponding to the plurality of surgical software applications that Dr. A may access (App 1, App 2, App 2, and App 4). The home screen 608 also includes a "Settings" icon to allow the user to view and modify various settings.

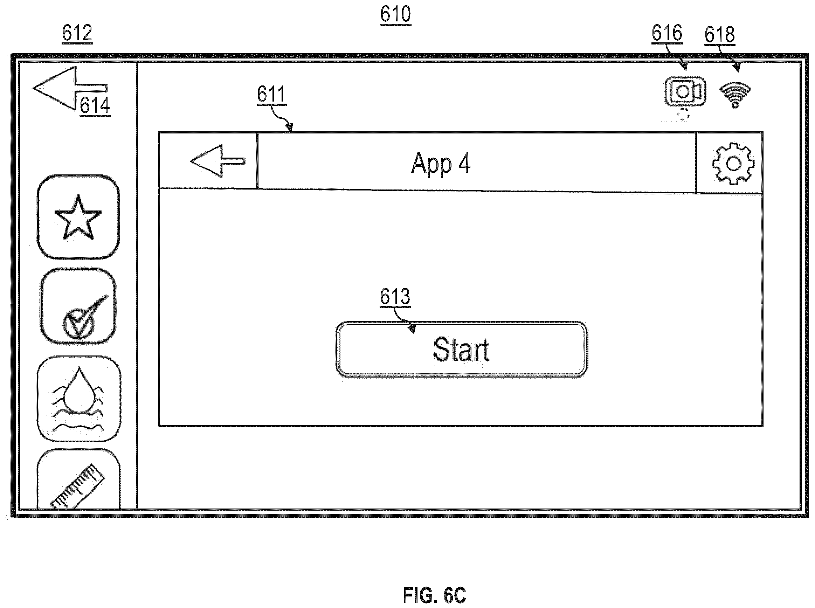

Upon a user selection of a surgical software application from the home screen 608 in FIG. 6B, the device displays a user interface 610 as shown in FIG. 6C. The user interface 610 includes an application panel 611 for displaying the graphical user interfaces of the selected surgical software application. The selected surgical software application may output data that can be displayed via the application panel 611 and/or via one or more other displays in the surgical environment. The application panel 611 can be used to display, for example, settings that are specific to the selected surgical software application, performance data, patient health data, readings from medical devices (e.g., pump pressure), user interface controls, outputs from the surgical software application (e.g., predictions or processed image data from machine-learning models), and the like. In the depicted example, the application panel 611 displays a user interface control 613 for starting a functionality of the selected surgical software application, App 4.

The contents of the application panel 611 may be defined by the surgical software application. As such, at least some of the contents of the application panel 611 may be different depending on which surgical software application has been selected from the home screen 608. The contents of the application panel 611 upon selection of a surgical software application from the home screen 608 may be different depending on the user and/or type of user that is logged into the device. Thus, different users and/or different types of users may be provided with different contents of the application panel 611 upon selection of a given surgical software application from the home screen 608. For example, a given surgical software application may define different contents for the application panel 611 for an administrator than for a normal user, such as a surgeon. The contents of the application panel 611 for an administrator may be directed to administrative functions, such as configuring hardware settings, and the contents of the application panel 611 for a surgeon may be directed to the functionality of the surgical software application. Since the contents of the application panel 611 may be defined by the surgical software application, some surgical software applications may provide different contents for different users and/or user types and other surgical software applications may not.

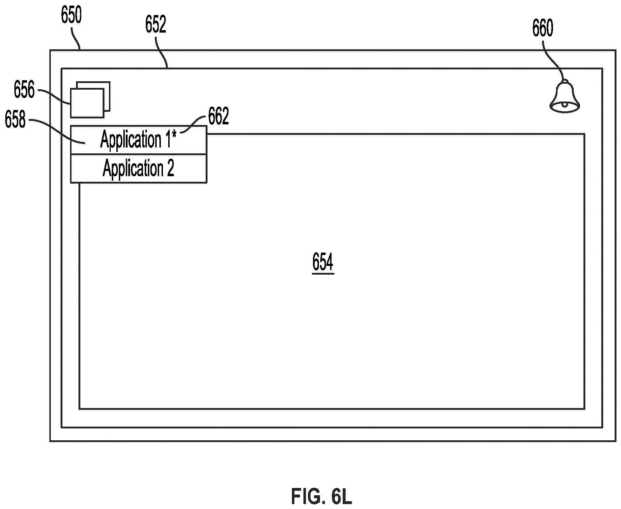

A surgical software application may additionally, or alternatively, define at least some of the contents of a user interface that is displayed on an external display that is communicatively connected to the device (e.g., device 102 or device 200). The external display may be, for example, a boom mounted display that is conveniently positioned for observation by a user, such as a surgeon, during a surgical procedure. FIG. 6L illustrates an exemplary external display 650 that can be controlled by the device. A graphical user interface 652 may be displayed on the display and may include an application panel 654 for displaying a graphical user interface defined by the selected surgical software application. A user may interact with the graphical user interface 652 differently than with the graphical user interface 610 of the device. For example, both the device and the external display 650 may be touchscreens such that one or more users can interact with graphical user interface 652 of the external display 650 or graphical user interface 610 of the device via touches to the respective touchscreen.

The graphical user interface 652 may include an application switching icon 656 that a user may use to switch to a different surgical software application (e.g., "Application 1" or "Application 2") that is running on the device, thereby selecting which graphical user interface is displayed in application panel 654. Selection of the application switching icon 656 by a user may result in display of a list 658 of running surgical software applications from which a user may select to change what is shown in application panel 654.

The graphical user interface 652 may include a notification icon 660 that may provide a user with notifications associated with the surgical software application(s) running on the device and/or the device itself. A user may select the notification icon 660 to obtain any notifications. The notification icon 660 and any notification associated with the notification icon 660 may be controlled by the device, not by the surgical software application that controls the contents of the application panel 654. As such, notifications generated by processes other than processes of the surgical software application may trigger display of the notification icon 660 regardless of which surgical software application is in control of the contents of the application panel 654.

The appearance of the notification icon 660 may be different for different types of notifications. For example, higher priority notifications (such as notifications associated with patient safety) may be indicated by a red notification icon 660 and lower priority notifications (such as notifications not associated with patient safety) may be indicated by a yellow notification icon 660. Additionally, or alternatively, the type of icon used for notification icon 660 may be different for different types of notifications. A user may dismiss a notification, which may result in removal of the notification icon 660 from the user interface 652. A notification icon providing similar functionality may be displayed on the display of the device (e.g., in user interface 610). Optionally, an indicator 662 may be provided in the list 658 of running surgical software applications that may indicate which surgical software application originated a notification.

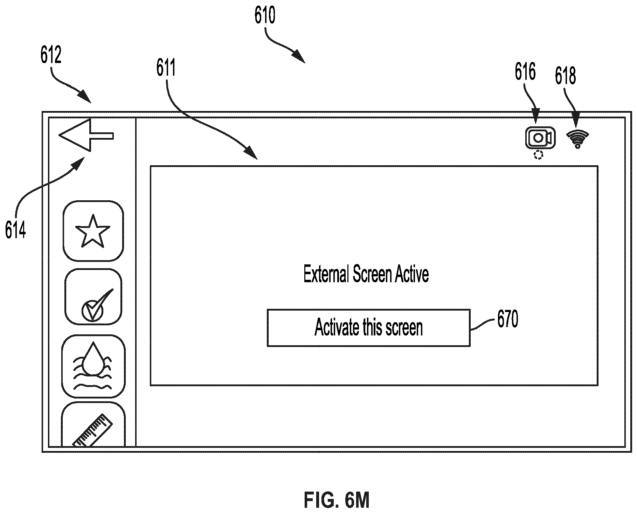

A given surgical software application may provide different graphical user interfaces for display on external display 650 than for display on the device. Optionally, the surgical software application may provide at least one graphical user interface that is displayable on the device and on the external display 650. For example, an application startup graphical user interface and/or a settings graphical user interface may be displayable on the device and the external display 650. Since a user may provide independent user input for the device and for the external display 650 (e.g., they are both touchscreens), display of the same graphical user interference on both the device and the external display 650 may lead to user input collisions whereby conflicting user inputs are provided to the device and the external display 650 at the same time (e.g., a surgeon interacts with the external display 650 at the same time that a staff member interacts with the device). To avoid this risk, the device may be configured to disable interaction with the graphical user interface of either the device or the external display 650 when the same graphical user interface is intended for display on both the device and the external display 650. For example, a given graphical user interface of a surgical software application may be displayed on the device, a user may cause that same graphical user interface to be displayed on the external display 650, and the graphical user interface on the device may be disabled, providing control of the graphical user interface solely to the external display 650. A warning may be provided indicating that the graphical user interface on the device has been disabled. An example of this is illustrated in FIG. 6M in which the graphical user interface shown in the application panel 611 is no longer shown and a warning is shown in its place indicating that the external screen is active. A user input feature 670 may be provided that enables a user to switch control of the graphical user interface from the external display 650 to the device. Upon selection of the user input feature 670, the graphical user interface may become active within the application panel 611 and the same disabling of the graphical user interface of the external display 650 and display of the warning and user input feature 670 may occur on the external display 650. The user may switch back to the external display 650 via selection of the user input feature 670 displayed on the external display 650.

The user interface 610 further includes a task bar 612. The task bar 612 can display a list of surgical software applications. The list of surgical software applications can include one or more currently executed surgical software applications, one or more surgical software applications that the user can access, one or more surgical software applications that are more commonly used by the user, or any combination thereof. The order of the surgical software applications in the task bar 612 may be based on installation time, launching time, usage, user preference, or any combination thereof. Upon user selection of a surgical software application in task bar 612, the application panel 611 can display the user interfaces of the selected surgical software application. The task bar 612 further includes at least one user interface control element 614, which may be used to exit a given surgical software application that is running. For examples, the user interface control element 614 may be used for returning to the home screen 608 of applications in FIG. 6B. The user interface control element 614 may be used to force quit a surgical software application, such as if the surgical software application is frozen.

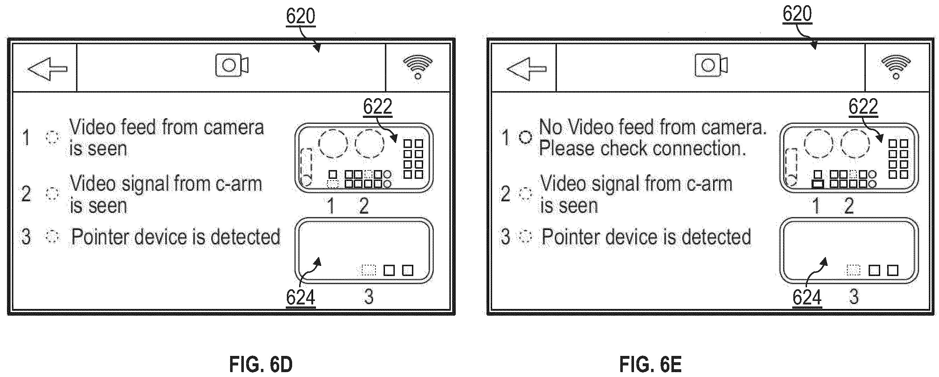

The user interface 610 further includes an icon 616 for viewing hardware components connected to the device and an icon 618 for presenting available networks that the device may connect to. FIGS. 6D-E illustrate an exemplary user interface 620 for displaying hardware components connected to the device. The user interface 620 may be displayed upon a user selection of the icon 616 in FIG. 6C. With reference to FIG. 6D, the user interface 620 includes a front panel representation 624 and a back panel representation 622. The front panel representation 624 shows the ports located on the front panel of the device and further indicates the corresponding status of the ports via visual cues (e.g., colors). The back panel representation 622 shows the ports located on the back panel of the device and further indicates the corresponding status of the ports via visual cues (e.g., colors). FIG. 6D further includes textual explanations of the status of the various ports of the device. In the depicted example, FIG. 6D includes textual explanations of three ports, which are labelled as 1, 2, and 3 in the front panel representation 624 and the back panel representation 622. In FIG. 6E, the user interface indicates that no data is received from port 1 via text and via the back panel representation 622.

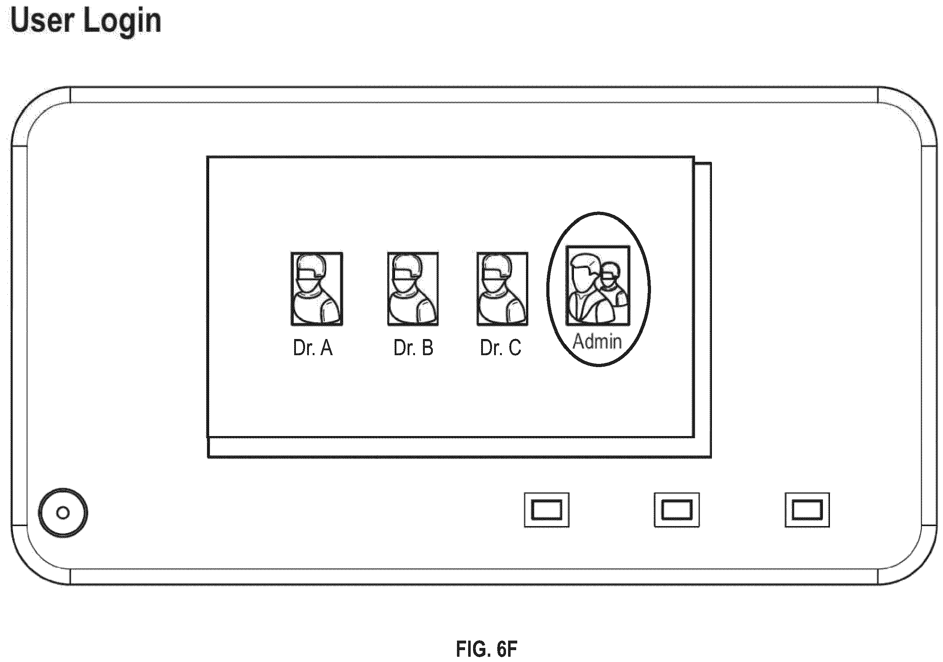

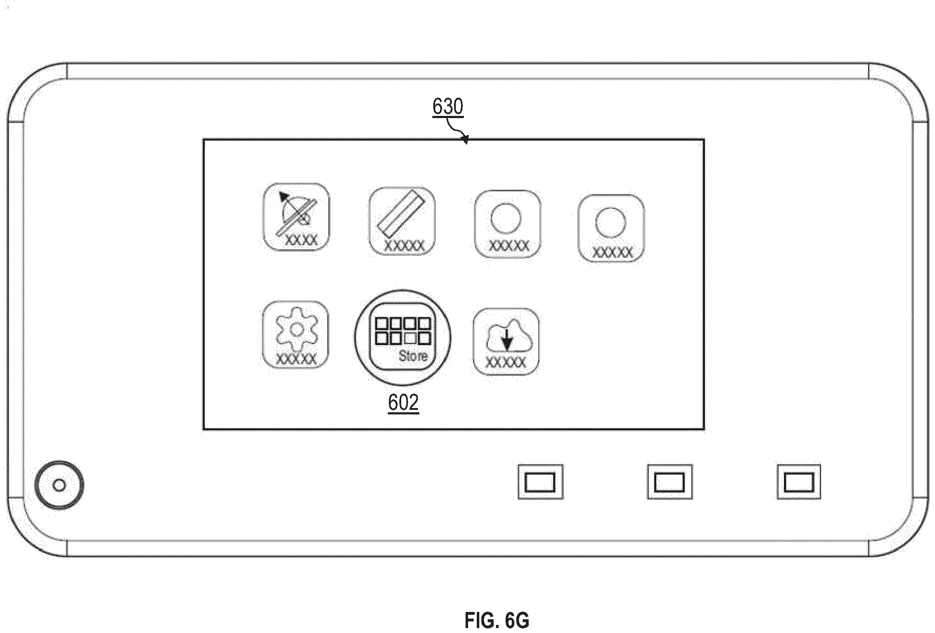

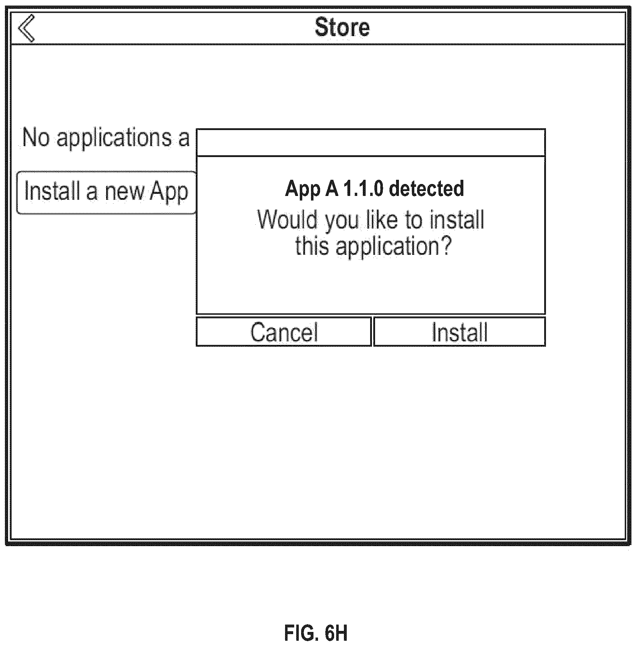

FIG. 6F illustrates that a user selects the user account corresponding to "Admin." In response to the selection, an administrator home screen 630 in FIG. 6G can be shown. The home screen 630 can include an icon 602 for a Store application. The Store application allows the user to acquire additional surgical software applications in his or her account or manage installed applications, as described below with reference to FIGS. 6H-K.

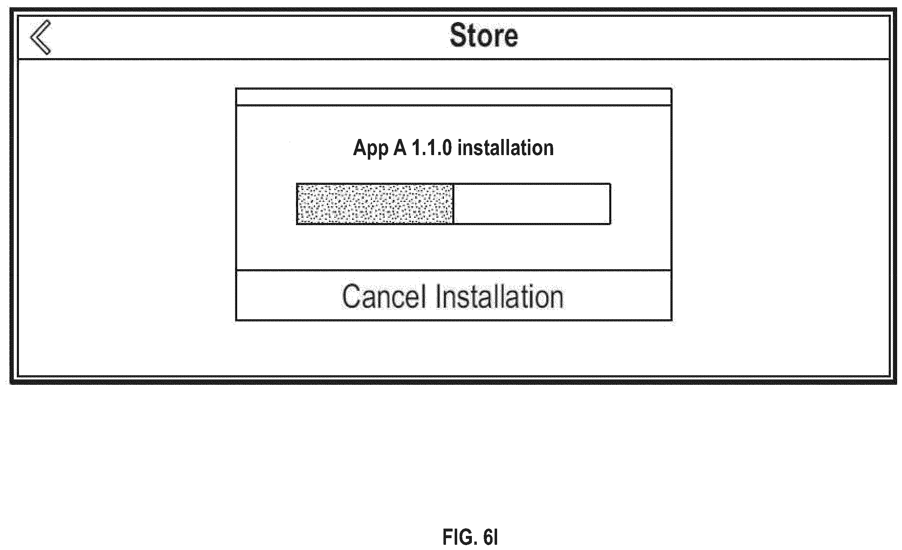

FIGS. 6H and 6I illustrate exemplary user interfaces for installing a surgical software application via a local device, in accordance with some examples. The device can be connected to a local device (e.g., via a USB connection) and detect that the local device provides new software applications. As shown in FIG. 6H, the device can automatically indicate that a new software application is detected and ask whether the user would like to install the new software application. FIG. 6I illustrates an exemplary user interface for indicating the progress of an ongoing installation.

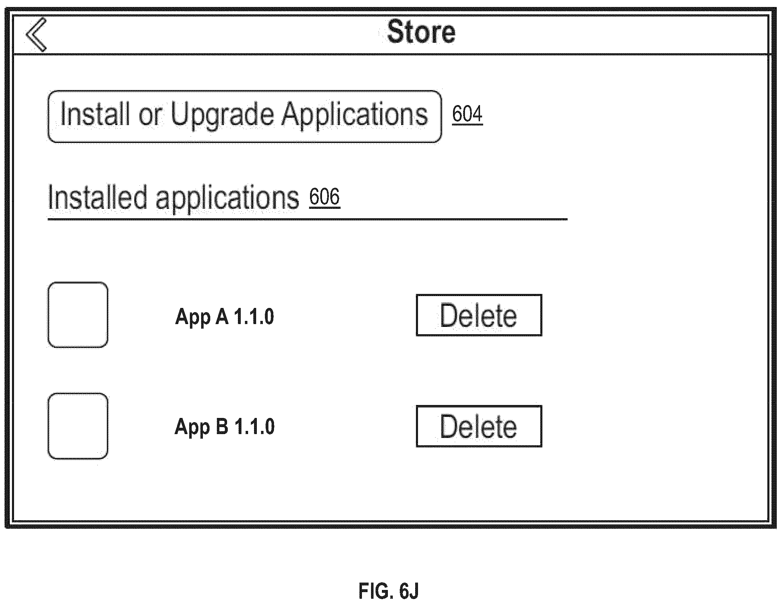

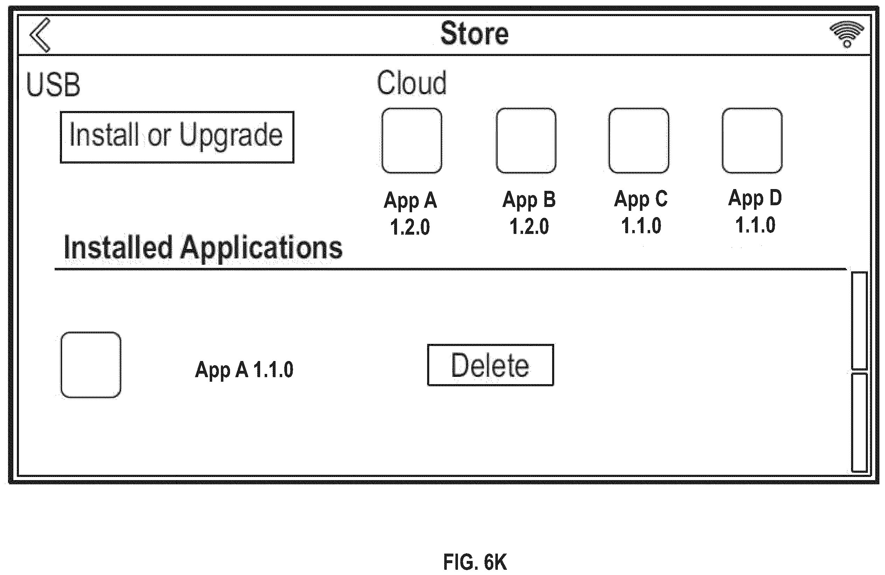

FIG. 6J illustrates an exemplary user interface of the Store application. As shown, the user interface includes a user interface control element 604 for installing and upgrading software applications. The user interface also displays a list 606 of installed software applications and allows the user to remove those software applications. Upon selection of user interface control element 604 in FIG. 6J, the device can display a user interface as shown FIG. 6K to provide a plurality of new applications that the user may install or upgrade to that are available from a remote device (e.g., on the cloud).

### Cooling Ducts

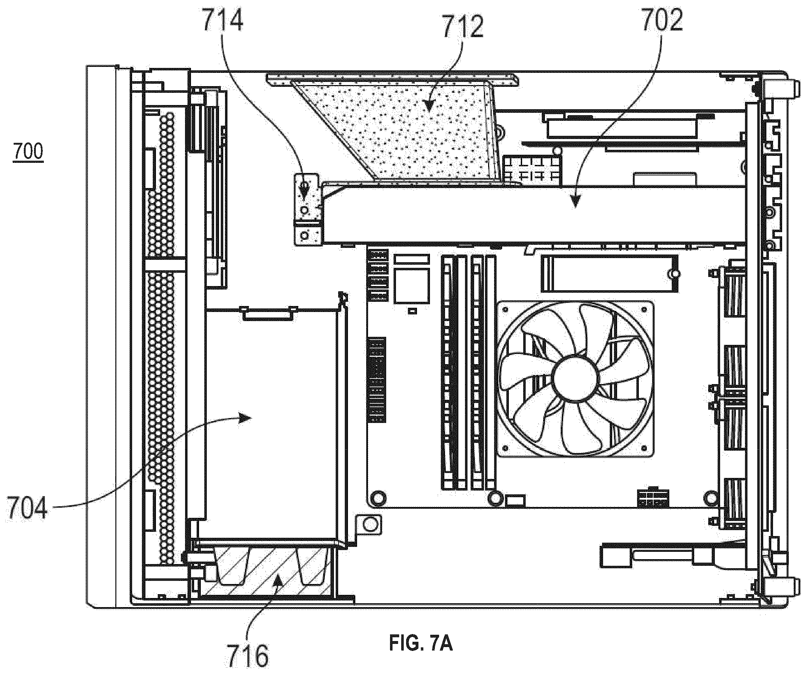

An exemplary device (e.g., device 200) for providing surgical software applications may include one or more cooling ducts for managing the temperature of various hardware components. FIG. 7A illustrates exemplary cooling ducts, in accordance with some examples. FIG. 7A depicts a device 700, which may be any of the devices described herein for providing surgical software applications. The device 700 includes a GPU 702 and a power supply unit ("PSU") 704. Further, a GPU duct 712 is positioned between the chassis of the device 700 and the GPU device 702. The GPU duct 712 includes a GPU support component 714 to secure the GPU 702 in place and provide structural support (e.g., such that the device can be transported with the GPU installed in the chassis without causing damage to the GPU). The GPU duct 712 provides a passage for air flow to help direct cooler air from outside the chassis to the GPU 702 and dissipate heat generated by the GPU 702. Further, a PSU duct 716 is positioned between the chassis of the device and the PSU 704. The PSU duct 716 provides a passage for air flow to help direct cooler air from outside the chassis and dissipate heat generated by the PSU 704.

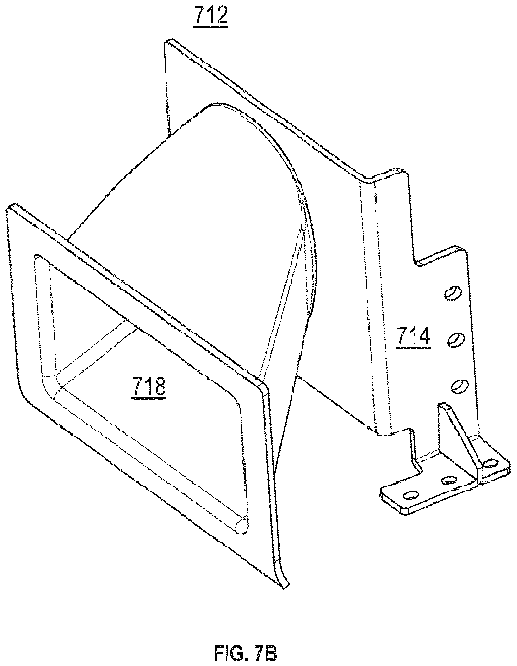

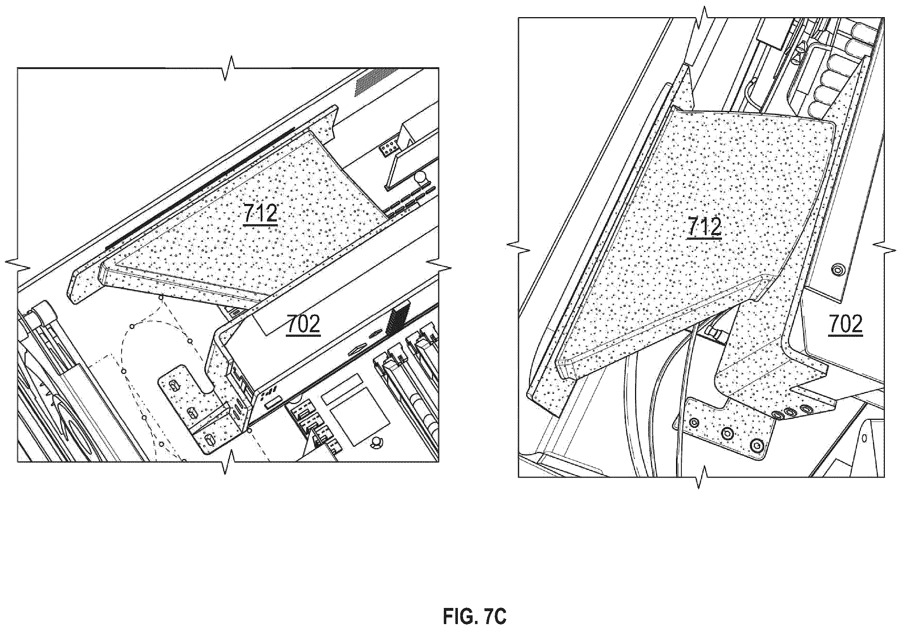

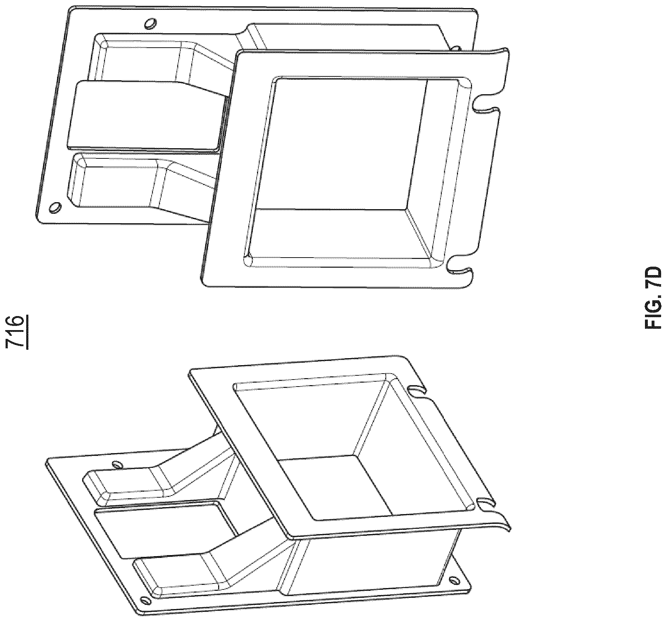

FIG. 7B illustrates an exemplary GPU duct 712, in accordance with some examples. As shown, the GPU duct 712 includes a GPU support component 714 and an air vent 718. FIG. 7C provides additional views showing how the GPU duct 712 is positioned to provide structural support for the adjacent GPU 702. FIG. 7D provides additional views of an exemplary PSU duct 716, in accordance with some examples. As shown, the PSU duct includes an air vent 718 to provide a passage for air flow to help direct cooler air from outside the chassis and dissipate heat generated by the PSU.

The GPU duct 712 and the PSU duct 716 may be manufactured using 3D printing techniques. Manufacturing the cooling ducts using 3D printing techniques can be more cost-efficient than using molding techniques, allow for generation of single pieces having complex geometries, and allow for quick updates to the designs due to changes to the PSU or GPU units. Accordingly, the dimensions and shapes of the GPU duct 712 and the PSU duct 716 can be modified to be adapted to support different types of GPUs and PSUs to dissipate heat and reduce vibration, thus reducing the need for dedicated support brackets.

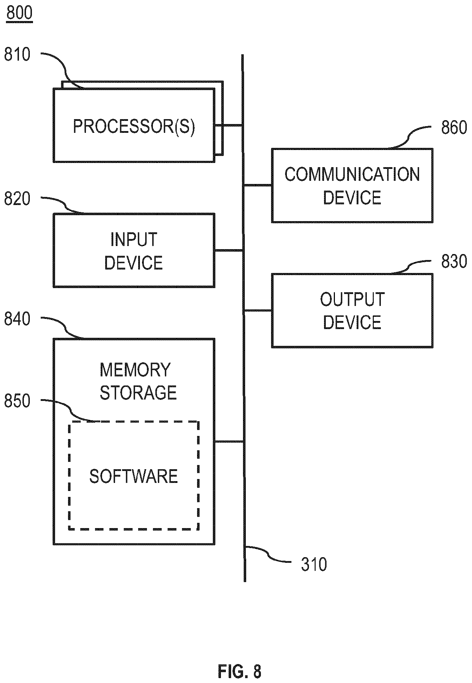

FIG. 8 illustrates an example of a computing device in accordance with one embodiment. The operations described above with reference to FIG. 2 are optionally implemented by components depicted in FIG. 8. Device 800 can be a host computer connected to a network. Device 800 can be a client computer or a server. As shown in FIG. 8, device 800 can be any suitable type of microprocessor-based device, such as a personal computer, workstation, server or handheld computing device (portable electronic device) such as a phone or tablet. The device can include, for example, one or more of processor 810, input device 820, output device 830, storage 840, and communication device 860. Input device 820 and output device 830 can generally correspond to those described above, and can either be connectable or integrated with the computer.

Input device 820 can be any suitable device that provides input, such as a touch screen, keyboard or keypad, mouse, or voice-recognition device. Output device 830 can be any suitable device that provides output, such as a touch screen, haptics device, or speaker.

Storage 840 can be any suitable device that provides storage, such as an electrical, magnetic or optical memory including a RAM, cache, hard drive, or removable storage disk. Communication device 860 can include any suitable device capable of transmitting and receiving signals over a network, such as a network interface chip or device. The components of the computer can be connected in any suitable manner, such as via a physical bus or wirelessly.

Software 850, which can be stored in storage 840 and executed by processor 810, can include, for example, the programming that embodies the functionality of the present disclosure (e.g., as embodied in the devices as described above).

Software 850 can also be stored and/or transported within any non-transitory computer-readable storage medium for use by or in connection with an instruction execution system, apparatus, or device, such as those described above, that can fetch instructions associated with the software from the instruction execution system, apparatus, or device and execute the instructions. In the context of this disclosure, a computer-readable storage medium can be any medium, such as storage 840, that can contain or store programming for use by or in connection with an instruction execution system, apparatus, or device.

Software 850 can also be propagated within any transport medium for use by or in connection with an instruction execution system, apparatus, or device, such as those described above, that can fetch instructions associated with the software from the instruction execution system, apparatus, or device and execute the instructions. In the context of this disclosure, a transport medium can be any medium that can communicate, propagate or transport programming for use by or in connection with an instruction execution system, apparatus, or device. The transport readable medium can include, but is not limited to, an electronic, magnetic, optical, electromagnetic or infrared wired or wireless propagation medium.

Device 800 may be connected to a network, which can be any suitable type of interconnected communication system. The network can implement any suitable communications protocol and can be secured by any suitable security protocol. The network can comprise network links of any suitable arrangement that can implement the transmission and reception of network signals, such as wireless network connections, T8 or T8 lines, cable networks, DSL, or telephone lines.

Device 800 can implement any operating system suitable for operating on the network. Software 850 can be written in any suitable programming language, such as C, C++, Java or Python. In various embodiments, application software embodying the functionality of the present disclosure can be deployed in different configurations, such as in a client/server arrangement or through a Web browser as a Web-based application or Web service, for example.

### EMBODIMENTS

The disclosure will now further describe the following numbered embodiments. It will be appreciated that some or all of the embodiments summarize aspects of the disclosure as provided in the description and the figures. Accordingly, the embodiments are also to be read in connection with the preceding paragraphs and the appended figures, and do not limit the disclosure. The features and preferences as described hereinabove apply also to the following embodiments.
Embodiment 1: A device for providing a plurality of surgical software applications, comprising:
   one or more processors;
   a plurality of surgical software applications for processing surgical data, wherein the plurality of surgical software applications are configured to be executed by the one or more processors, and wherein the plurality of surgical software applications include a plurality of sample input datasets corresponding to the plurality of surgical software applications; and
   one or more operating system programs for testing and providing the plurality of surgical software applications, wherein the one or more operating system programs are configured to be executed by the one or more processors, and wherein the one or more operating system programs include instructions for:
      receiving one or more user inputs indicative of one or more surgical software applications of the plurality of surgical software applications;
      retrieving one or more sample input datasets corresponding to the one or more surgical software applications from the plurality of sample input datasets;
      executing, based on the one or more sample input datasets, the one or more surgical software applications in a simulated environment;
      evaluating the execution of the one or more surgical software applications; and
      outputting, based on the evaluation of the execution of the one or more surgical software applications, a recommendation related to the one or more surgical software applications.
Embodiment 2: The device of embodiment 1, wherein a sample input dataset of the plurality of sample input datasets comprises: image data, textual data, audio data, simulated user input data, or any combination thereof.
Embodiment 3: The device of embodiment 2, wherein the sample input dataset of the plurality of sample input datasets comprises: surgical video data, medical record data, case file data, speech data, or any combination thereof.
Embodiment 4: The device of embodiment 1, 2 or 3, wherein evaluating the execution of the one or more surgical software applications comprises: identifying a software crash event, a software hang event, a software lag event, a failure-to-launch event, an error, or any combination thereof.
Embodiment 5: The device of any of embodiments 1-4, wherein evaluating the execution of the one or more surgical software applications comprises: evaluating usage of one or more memories of the device or the one or more processors of the device.
Embodiment 6: The device of any of embodiments 1-5, wherein the one or more operating system programs further include instructions for:
   receiving a user request to launch a surgical software application of the plurality of surgical software applications;
   retrieving one or more contract files associated with the surgical software application, wherein the one or more contract files comprise one or more parameters;
   comparing a current state of the device with the one or more parameters in the one or more contract files; and
   determining, based on the comparison between the current state of the device with the one or more parameters in the one or more contract files, whether to launch the surgical software application.
Embodiment 7: A device for providing a plurality of surgical software applications, comprising:
   one or more processors;
   a plurality of surgical software applications for processing surgical data, wherein the plurality of surgical software applications are configured to be executed by the one or more processors; and
   one or more operating system programs for testing and providing the plurality of surgical software applications, wherein the one or more operating system programs are configured to be executed by the one or more processors, and wherein the one or more operating system programs include instructions for:
      receiving a user request to launch a surgical software application of the plurality of surgical software applications;
      retrieving one or more contract files associated with the surgical software application, wherein the one or more contract files comprise one or more parameters;
      comparing a current state of the device with the one or more parameters in the one or more contract files; and
      determining, based on the comparison between the current state of the device with the one or more parameters in the one or more contract files, whether to launch the surgical software application.
Embodiment 8: The device of embodiment 6 or 7, wherein the one or more operating system programs further include instructions for: in accordance with a determination to not launch the surgical software application, outputting a user notification.
Embodiment 9: The device of embodiment 6, 7 or 8, wherein the one or more parameters in the one or more contract files comprise a first set of parameters specifying an amount of resource required to execute the surgical software application.
Embodiment 10: The device of embodiment 9, wherein the first set of parameters is indicative of: a minimum memory requirement, a minimum core requirement, a minimum usage percentage, or any combination thereof.
Embodiment 11: The device of any of embodiments 6-10, wherein the one or more parameters in the one or more contract files comprise a second set of parameters specifying one or more hardware components required to execute the surgical software application.
Embodiment 12: The device of embodiment 11, wherein the one or more hardware components comprise: a central processing unit (CPU), a graphics processing unit (GPU), a capture card, an isolation port, a USB port, or any combination thereof.
Embodiment 13: The device of any of embodiments 6-12, wherein the one or more parameters in the one or more contract files comprise a third set of parameters specifying compatibility constraints of the surgical software application.
Embodiment 14: The device of embodiment 13, wherein the third set of parameters is indicative of: one or more software environments that the surgical software application is compatible with.
Embodiment 15: The device of any of embodiments 6-14, wherein the one or more parameters in the one or more contract files comprise a fourth set of parameters specifying a relative priority of the surgical software application.
Embodiment 16: The device of embodiment 15, wherein the relative priority is determined based on a relative risk level associated with a surgical procedure.
Embodiment 17: The device of embodiment 15 or 16, wherein the fourth set of parameters is indicative of: a priority value of the surgical software application and/or whether the surgical software application can run without a displayed graphical user interface.
Embodiment 18: The device of any of embodiment 1-17, wherein at least one surgical software application of the plurality of surgical software applications is subscription-based.
Embodiment 19: The device of any of embodiment 1-18, wherein the device further comprises a chassis and a temperature sensor inside the chassis, and wherein the one or more operating system programs further include instructions for:
   obtaining, via the temperature sensor, a temperature associated with the device;
   if the temperature exceeds a predefined temperature threshold, reducing an amount of power provided to the one or more processors.
Embodiment 20: The device of embodiment 19, wherein the one or more operating system programs further include instructions for: if the temperature exceeds the predefined temperature threshold, terminating an active surgical software application being executed by the one or more processors.
Embodiment 21: The device of embodiment 20, wherein the active surgical software application is selected based on a priority value associated with the active surgical software application and/or a last-used time associated with the active surgical software application.
Embodiment 22: The device of any of embodiments 19-21, wherein the device comprises a fan and wherein the one or more operating system programs further include instructions for: if the temperature exceeds the predefined temperature threshold, increasing a speed of the fan.
Embodiment 23: The device of any of embodiments 1-22, wherein the one or more operating system programs further include instructions for:
   receiving a user input indicative of a user identity;
   retrieving a user profile associated with the user identity; and
   configuring the device based on the user profile.
Embodiment 24: The device of any of embodiments 1-23, wherein the plurality of surgical software applications for processing surgical data are stored in one or more memories of the device.
Embodiment 25: The device of any of embodiments 1-24, wherein the plurality of surgical software applications for processing surgical data are stored in one or more remote devices.
Embodiment 26: A method for providing a plurality of surgical software applications, comprising:
   receiving, by one or more operating system programs of a device, one or more user inputs indicative of one or more surgical software applications of a plurality of surgical software applications of the device, wherein the plurality of surgical software applications include a plurality of sample input datasets corresponding to the plurality of surgical software applications;
   retrieving, by the one or more operating system programs of the device, one or more sample input datasets corresponding to the one or more surgical software applications from the plurality of sample input datasets;
   executing, by one or more processors of the device, based on the one or more sample input datasets, the one or more surgical software applications in a simulated environment;
   evaluating, by the one or more operating system programs of the device, the execution of the one or more surgical software applications; and
   outputting a recommendation related to the one or more surgical software applications.
Embodiment 27: The method of embodiment 26, wherein a sample input dataset of the plurality of sample input datasets comprises: image data, textual data, audio data, simulated user input data, or any combination thereof.
Embodiment 28: The method of embodiment 27, wherein the sample input dataset of the plurality of sample input datasets comprises: surgical video data, medical record data, case file data, speech data, or any combination thereof.
Embodiment 29: The method of any of embodiments 26-28, wherein evaluating the execution of the one or more surgical software applications comprises: identifying a software crash event, a software hang event, a software lag event, a failure-to-launch event, an error, or any combination thereof.
Embodiment 30: The method of any of embodiments 26-29, wherein evaluating the execution of the one or more surgical software applications comprises: evaluating usage of one or more memories of the device or the one or more processors of the device.
Embodiment 31: The method of any of embodiments 26-30, further comprising:
   receiving a user request to launch a surgical software application of the plurality of surgical software applications;
   retrieving one or more contract files associated with the surgical software application, wherein the one or more contract files comprise one or more parameters;
   comparing a current state of the device with the one or more parameters in the one or more contract files; and
   determining, based on the comparison between the current state of the device with the one or more parameters in the one or more contract files, whether to launch the surgical software application.
Embodiment 32: A method for providing a plurality of surgical software applications, comprising:
   receiving a user request to launch a surgical software application of the plurality of surgical software applications;
   retrieving one or more contract files associated with the surgical software application, wherein the one or more contract files comprise one or more parameters;
   comparing a current state of the device with the one or more parameters in the one or more contract files; and
   determining, based on the comparison between the current state of the device with the one or more parameters in the one or more contract files, whether to launch the surgical software application.
Embodiment 33: The method of embodiment 31 or 32, further comprising: in accordance with a determination to not launch the surgical software application, outputting a user notification.
Embodiment 34: The method of embodiment 31, 32 or 33, wherein the one or more parameters in the one or more contract files comprise a first set of parameters specifying an amount of resource required to execute the surgical software application.
Embodiment 35: The method of embodiment 34, wherein the first set of parameters is indicative of: a minimum memory requirement, a minimum core requirement, a minimum usage percentage, or any combination thereof.
Embodiment 36: The method of any of embodiments 31-35, wherein the one or more parameters in the one or more contract files comprise a second set of parameters specifying one or more hardware components required to execute the surgical software application.
Embodiment 37: The method of embodiment 36, wherein the one or more hardware components comprise: a central processing unit (CPU), a graphics processing unit (GPU), a capture card, an isolation port, a USB port, or any combination thereof.
Embodiment 38: The method of any of embodiments 31-37, wherein the one or more parameters in the one or more contract files comprise a third set of parameters specifying compatibility constraints of the surgical software application.
Embodiment 39: The method of embodiment 38, wherein the third set of parameters is indicative of: one or more software environments that the surgical software application is compatible with.
Embodiment 40: The method of any of embodiments 31-39, wherein the one or more parameters in the one or more contract files comprise a fourth set of parameters specifying a relative priority of the surgical software application.
Embodiment 41: The method of embodiment 40, wherein the relative priority is determined based on a relative risk level associated with a surgical procedure.
Embodiment 42: The method of embodiment 40 or 41, wherein the fourth set of parameters is indicative of: a priority value of the surgical software application and/or whether the surgical software application can run without a displayed graphical user interface.
Embodiment 43: The method of any one of embodiments 26-42, wherein at least one surgical software application of the plurality of surgical software applications is subscription-based.
Embodiment 44: The method of any one of embodiments 26-43, wherein the device further comprises a chassis and a temperature sensor inside the chassis, the method further comprising: obtaining, via the temperature sensor, a temperature associated with the device;
   if the temperature exceeds a predefined temperature threshold, reducing an amount of power provided to the one or more processors.
Embodiment 45: The method of embodiment 44, further comprising: if the temperature exceeds the predefined temperature threshold, terminating an active surgical software application being executed by the one or more processors.
Embodiment 46: The method of embodiment 45, wherein the active surgical software application is selected based on a priority value associated with the active surgical software application and/or a last-used time associated with the active surgical software application.
Embodiment 47: The method of any of embodiments 44-46, wherein the device comprises a fan, the method further comprising: if the temperature exceeds the predefined temperature threshold, increasing a speed of the fan.
Embodiment 48: The method of any of embodiments 26-47, further comprising:
   receiving a user input indicative of a user identity;
   retrieving a user profile associated with the user identity; and
   configuring the device based on the user profile.
Embodiment 49: The method of any of embodiments 26-48, wherein the plurality of surgical software applications for processing surgical data are stored in one or more memories of the device.
Embodiment 50: The method of any of embodiments 26-49, wherein the plurality of surgical software applications for processing surgical data are stored in one or more remote devices.
Embodiment 51: A non-transitory computer-readable storage medium storing one or more programs, the one or more programs comprising instructions, which when executed by one or more processors of an electronic device, cause the electronic device to perform the method of any of embodiments 26-50.
Embodiment 52: A computer program product comprising software code portions, which when executed by one or more processors of an electronic device, cause the electronic device to perform the method of any of embodiments 26-50.

The foregoing description, for the purpose of explanation, has been described with reference to specific examples or aspects. However, the illustrative discussions above are not intended to be exhaustive or to limit the invention to the precise forms disclosed. For the purpose of clarity and a concise description, features are described herein as part of the same or separate variations; however, it will be appreciated that the scope of the disclosure includes variations having combinations of all or some of the features described. Many modifications and variations are possible in view of the above teachings. The variations were chosen and described in order to best explain the principles of the techniques and their practical applications. Others skilled in the art are thereby enabled to best utilize the techniques and various variations with various modifications as are suited to the particular use contemplated.

Although the disclosure and examples have been fully described with reference to the accompanying figures, it is to be noted that various changes and modifications will become apparent to those skilled in the art. Such changes and modifications are to be understood as being included within the scope of the disclosure and examples as defined by the claims. Finally, the entire disclosure of the patents and publications referred to in this application are hereby incorporated herein by reference.

For the purpose of clarity and a concise description, features are described herein as part of the same or separate examples; however, it will be appreciated that the scope of the disclosure includes examples having combinations of all or some of the features described.

## Claims

1. A device for providing a plurality of surgical software applications, comprising: one or more processors;
a plurality of surgical software applications for processing surgical data,
wherein the plurality of surgical software applications are configured to be executed by the one or more processors; and
one or more operating system programs for testing and providing the plurality of surgical software applications,
wherein the one or more operating system programs are configured to be executed by the one or more processors, and
wherein the one or more operating system programs include instructions for:
receiving a user request to launch a surgical software application of the plurality of surgical software applications;
retrieving one or more contract files associated with the surgical software application, wherein the one or more contract files comprise one or more parameters;
comparing a current state of the device with the one or more parameters in the one or more contract files; and
determining, based on the comparison between the current state of the device with the one or more parameters in the one or more contract files, whether to launch the surgical software application.

2. The device of claim 1, wherein the one or more operating system programs further include instructions for: in accordance with a determination to not launch the surgical software application, outputting a user notification.

3. The device of claim 1 or claim 2, wherein the one or more parameters in the one or more contract files comprise a first set of parameters specifying an amount of resource required to execute the surgical software application, wherein the first set of parameters is e.g. indicative of: a minimum memory requirement, a minimum core requirement, a minimum usage percentage, or any combination thereof.

4. The device of claim 1, 2 or 3, wherein the one or more parameters in the one or more contract files comprise a second set of parameters specifying one or more hardware components required to execute the surgical software application, wherein the one or more hardware components e.g. comprise: a central processing unit (CPU), a graphics processing unit (GPU), a capture card, an isolation port, a USB port, or any combination thereof.

5. The device of any of claims 1-4, wherein the one or more parameters in the one or more contract files comprise a third set of parameters specifying compatibility constraints of the surgical software application, wherein the third set of parameters is e.g. indicative of: one or more software environments that the surgical software application is compatible with.

6. The device of any of claims 1-5, wherein the one or more parameters in the one or more contract files comprise a fourth set of parameters specifying a relative priority of the surgical software application, wherein the relative priority is e.g. determined based on a relative risk level associated with a surgical procedure.

7. The device of claim 6, wherein the fourth set of parameters is indicative of: a priority value of the surgical software application and/or whether the surgical software application can run without a displayed graphical user interface.

8. The device of any of the preceding claims, wherein at least one surgical software application of the plurality of surgical software applications is subscription-based.

9. The device of any of the preceding claims, wherein the device further comprises a chassis and a temperature sensor inside the chassis, and wherein the one or more operating system programs further include instructions for:
obtaining, via the temperature sensor, a temperature associated with the device;
if the temperature exceeds a predefined temperature threshold, reducing an amount of power provided to the one or more processors.

10. The device of claim 9, wherein the one or more operating system programs further include instructions for: if the temperature exceeds the predefined temperature threshold, terminating an active surgical software application being executed by the one or more processors, wherein the active surgical software application is e.g. selected based on a priority value associated with the active surgical software application and/or a last-used time associated with the active surgical software application.

11. The device o claim 9 or 10, wherein the device comprises a fan and wherein the one or more operating system programs further include instructions for: if the temperature exceeds the predefined temperature threshold, increasing a speed of the fan.

12. The device of any of the preceding claims, wherein the one or more operating system programs further include instructions for:
receiving a user input indicative of a user identity;
retrieving a user profile associated with the user identity; and
configuring the device based on the user profile.

13. The device of any one of the preceding claims, wherein the plurality of surgical software applications for processing surgical data are stored in one or more memories of the device and/or in one or more remote devices.

14. A method for providing a plurality of surgical software applications, comprising: receiving a user request to launch a surgical software application of the plurality of surgical software applications;
retrieving one or more contract files associated with the surgical software application, wherein the one or more contract files comprise one or more parameters;
comparing a current state of the device with the one or more parameters in the one or more contract files; and
determining, based on the comparison between the current state of the device with the one or more parameters in the one or more contract files, whether to launch the surgical software application.

15. A computer program product comprising software code portions, which when executed by one or more processors of an electronic device, cause the electronic device to perform the methods of claim 14.
